# EUROPÄISCHE PATENTSCHRIFT

(11) **EP 3 068 304 B1**
(45) Veröffentlichungstag und Bekanntmachung des Hinweises auf die Patenterteilung: **09.08.2017**
(21) Anmeldenummer: 14825086.3
(22) Anmeldetag: 13.11.2014
(51) Int. Cl.: A61B 5/154, B01D 21/26, B04B 5/04, A61B 5/15, A61B 5/153, G01N 33/49, A61B 10/00, B01L 3/00

(54) **AUFNAHMEVORRICHTUNG, VERFAHREN ZUM BEREITSTELLEN DERSELBEN SOWIE VERFAHREN ZUM TRENNEN EINES GEMISCHES**
RECEPTACLE DEVICE, METHOD FOR PROVIDING THE SAME AND METHOD FOR SEPARATING A MIXTURE
DISPOSITIF DE RÉCEPTION, PROCÉDÉ DE PRODUCTION DE CELUI-CI ET PROCÉDÉ DE SÉPARATION D'UN MÉLANGE

(30) Priorität: 14.11.2013 AT 507592013
(43) Veröffentlichungstag der Anmeldung: 21.09.2016
(73) Patentinhaber: Greiner Bio-One GmbH, 4550 Kremsmünster (AT)
(72) Erfinder: BAUER, Christian, A-4550 Kremsmünster (AT)
(74) Vertreter: Burger, Hannes
(86) Internationale Anmeldenummer: PCT/AT2014/050273
(87) Internationale Veröffentlichungsnummer: WO 2015/070273

(56) Entgegenhaltungen:
- EP-A1- 0 001 200
- EP-A2- 0 017 127
- WO-A1-02/073190
- US-A- 3 508 653
- US-A- 4 235 725
- US-A1- 2010 288 694

## Beschreibung

Die Erfindung betrifft eine Aufnahmevorrichtung zum Trennen eines Gemisches, insbesondere von Blut, wie diese im Anspruch 1 beschrieben ist. Des Weiteren Betrifft diese Erfindung aber auch noch ein Verfahren zum Bereitstellen einer derartigen Aufnahmevorrichtung sowie ein Verfahren zum Trennen eines Gemisches, insbesondere von Blut, in seine leichtere Phase mit geringerer Dichte und seine schwerere Phase mit einer dazu größeren Dichte, wie dies in den Ansprüchen 10, 11, 12 und 14 beschrieben ist.

Eine gattungsgemäß ausgebildete Aufnahmevorrichtung zum Trennen von Blut, in eine leichtere Phase mit geringerer Dichte und in eine schwerere Phase mit einer dazu größeren Dichte ist aus der WO 02/073190 A1 bekannt geworden. Diese Aufnahmevorrichtung umfasst einen röhrchenförmig ausgebildeten Aufnahmebehälter, der an seinem ersten Ende offen und an seinem zweiten Ende mit einer Bodenwand verschlossen ausgebildet ist. Das offene erste Ende ist mit einem Verschlussstopfen verschlossen. Der Aufnahmebehälter weist eine Seitenwand mit einer Innenfläche und einer Außenfläche auf. Im Aufnahmeraum des Aufnahmebehälters ist ein aus einem elastisch verformbaren Werkstoff gebildetes Trennelement aufgenommen, dessen Dichte zwischen den zu trennenden Phasen liegt. Das Trennelement weist in seiner unverformten Ausgangsform eine überwiegend kugelig ausgebildete Raumform auf und ist vor dem Befüllen des Aufnahmeraums im Bereich des mit dem Boden verschlossenen zweiten Endes angeordnet. In dieser Position liegt das Trennelement in einer senkrecht bezüglich der Längsachse ausgerichteten Anlageebene an der Innenfläche der Seitenwand an. Ein Außendurchmesser des Trennelements ist in seiner unverformten Ausgangsform größer gewählt als eine Querschnittsabmessung des Aufnahmeraums in der Anlageebene. Das Trennelement ist an der Innenfläche der Seitenwand in einer vorgespannten Bereitschaftsposition positioniert daran gehalten.

Eine weitere gattungsgemäß ausgebildete Aufnahmevorrichtung mit einem darin angeordneten Trennelement beschreibt die EP 0 017 127 A2. Dabei wird das Trennelement entweder erst nach dem Befüllen des Aufnahmeraums mit dem zu trennenden Gemisch in das offene Ende des Aufnahmebehälters eingesetzt oder das Trennelement ist bereits am Verschlussstopfen gehalten. Das Trennelement kann hier auch unter Anderem eine Kugelform aufweisen. Während des Zentrifugationsvorgangs wird das Trennelement ausgehend vom offenen Ende hin in Richtung auf das mit dem Boden verschlossene Ende verstellt. Nach Beendigung des Zentrifugationsvorgangs ist das Trennelement zwischen den beiden zu trennenden Phasen des Gemischs angeordnet und trennt diese voneinander.

Eine weitere Aufnahmevorrichtung zum Trennen einer Flüssigkeitsprobe in ihre schwerere Phase und in ihre leichtere Phase ist aus der US 2010/0288694 A1 bekannt geworden. Der röhrchenförmig ausgebildete Aufnahmebehälter weist ausgehend von seinem offenen Ende einen ersten Teilabschnitt mit einer eine größere Abmessung aufweisenden Seitenwand als in dem zweiten daran in Richtung auf das verschlossene Ende anschließenden zweiten Teilabschnitt auf. Das Trennelement ist vor dem Beginn des Zentrifugationsvorgangs im ersten Teilabschnitt mit der größeren Innenabmessung angeordnet. Innerhalb des Trennelements ist ein Kanal vorgesehen, um die zu trennende Flüssigkeitsprobe ausgehend von dem mit einem Verschlusselement verschlossenen Ende in den Aufnahmeraum einbringen zu können.

Weitere Aufnahmevorrichtung zum Trennen einer Flüssigkeitsprobe in ihre schwerere Phase und in ihre leichtere Phase sind aus der EP 0 001 200 A1, der US 4,235,725 A und der US 3,508,653 A bekannt geworden.

So beschreibt die EP 0 001 200 A1 einen röhrchenförmig ausgebildeten Aufnahmebehälter, bei dem beide Enden mit einem Verschlusselement verschlossen sind. Das Trennelement ist während des Befüllvorgangs der Flüssigkeitsprobe bodenseitig angeordnet. Für den Zentrifugationsvorgang wird die Aufnahmevorrichtung gewendet, sodass das zuvor bodenseitige Ende das obenliegende Ende bildet. Das Trennelement weist zwei scheibenförmig ausgebildete und in Axialrichtung hintereinander angeordnete Dichtelemente auf, welche an der Innenfläche des Aufnahmebehälters rundum dichtend anliegen. Während des Zentrifugationsvorgangs werden die Dichtelemente umgeformt, um so zwischen der Innenfläche des Aufnahmebehälters und den Dichtelementen einen Kanal auszubilden.

Die US 4,235,725 A beschreibt einen röhrchenförmig ausgebildeten Aufnahmebehälter, bei dem das Trennelement durch ein Gel gebildet ist. Vor Beginn des Einfüllvorgangs befindet sich das Trenngel in dem mit einer Bodenwand verschlossenen Ende des Aufnahmebehälters und wird im Zuge des Zentrifugationsvorgangs zwischen die zu trennenden Phasen der Flüssigkeitsprobe, nämlich dem Blut, verlagert.

Aus der US 3,508,653 A bzw. der DE 1 806 196 A ist eine Aufnahmevorrichtung zum Trennen von Flüssigkeiten, insbesondere von Blut, in seine leichtere Phase mit geringerer Dichte und seine schwerere Phase mit einer dazu größeren Dichte bekannt geworden. Die Aufnahmevorrichtung umfasst einen Aufnahmebehälter, der in Richtung einer Längsachse voneinander distanzierte erste und zweite Enden aufweist, wobei das erste Ende offen und das zweite Ende mit einem Boden verschlossen ist. Zwischen dem ersten und dem zweiten Ende erstreckt sich eine Seitenwand mit einer Innenfläche und einer Außenfläche, wobei die Seitenwand und der Boden einen Aufnahmeraums umgrenzen. Weiters ist im Aufnahmeraums ein als Kolben ausgebildetes Trennelement angeordnet, welches vollständig aus einem elastisch verformbaren Werkstoff gebildet ist. Mit einer abnehmbaren Verschlusseinheit ist das offene Ende des Aufnahmebehälters verschlossen. Der Kolben ist vor dem Beginn der Separation lösbar an einem Dichtelement der Verschlusseinheit positioniert gehalten.

Eine andere Trennvorrichtung zum Trennen von Flüssigkeiten, insbesondere von Blut, in seine leichtere Phase mit geringerer Dichte und seine schwerere Phase mit einer dazu größeren Dichte ist aus der JP 56-168847 A bekannt geworden. Dabei ist im Aufnahmebehälter als Trennelement eine eine sphärische Raumform aufweisende Hülle aus einer dünnen Folie angeordnet, deren Innenraum mit einem Gel befüllt ist.

Die EP 1 005 909 B1 beschreibt eine Anordnung zum Separieren einer Fluidprobe in eine Phase mit höherer relativer Dichte und eine Phase mit geringerer relativer Dichte. Die Aufnahmevorrichtung umfasst ein Röhrchen mit einem offenen Ende und einem mit einem Boden verschlossen Ende. Zwischen den beiden Enden erstreckt sich eine Seitenwand mit einer Innenfläche und einer Außenfläche. Mit einem Verschluss ist das offene Ende des Röhrchens verschlossen. Im Röhrchen ist eine elastisch verformbare Auskleidung angeordnet, wobei diese elastisch aufweitbar ausgebildet ist. Weiters ist im Aufnahmeraums ein kugelförmiger Dichtungskörper angeordnet, welcher im nicht aufgeweiteten Zustand der Auskleidung an dieser anliegt. Der Dichtungskörper ist aus einem starren, thermoplastischen Material gebildet. Beim Separationsvorgang wird die Auskleidung elastisch verformt, wodurch der Dichtungskörper freigegeben wird und durch seine gewählte Dichte zwischen den beiden zu trennenden Phase auf der Phase mit der größeren Dichte aufschwimmt.

Weitere Trennvorrichtungen, bei denen das Trennelement mit einem bei Kontakt mit Flüssigkeit aufquellendem Material versehen bzw. beschichtet ist, sind auch bereits bekannt geworden. Als Beispiel sei hier die EP 0 744 026 B1 bzw. die DE 695 24 063 T2 angeführt. Diese sind in der Herstellung kostenintensiv und bei einigen Anwendungsfällen nicht ausreichend sicher in der Trennung.

Der vorliegenden Erfindung liegt die Aufgabe zugrunde, eine Aufnahmevorrichtung zu schaffen, welche kostengünstig in der Herstellung ist und dabei mit wenigen benötigten Bauteilen trotzdem eine sichere Trennung der voneinander separierten Phasen gewährleistet. Darüber hinaus soll aber auch ein Verfahren zur Bereitstellung einer derartigen Aufnahmevorrichtung angegeben werden, welches eine einfache und kostengünstige Herstellung ermöglicht. Des Weiteren soll aber auch ein Verfahren zum Separieren eines Gemisches unter Verwendung einer derartigen Aufnahmevorrichtung angegeben werden, welches eine einwandfreie Trennung der voneinander separierten Phasen auch über eine längere Lagerdauer sicherstellt.

Eine Aufgabe der Erfindung wird durch die Merkmale des Anspruchs 1 gelöst. Der sich durch die Merkmale des Anspruchs 1 ergebende Vorteil liegt darin, dass dadurch auch bei eingesetztem Trennelement im Bereich des Bodens auch der zwischen dem Trennelement und dem Boden ausgebildete Teilraum des Aufnahmeraums gemeinsam mit dem darüber angeordneten Teilraum auf einen gegenüber dem Umgebungsdruck liegenden Druck abgesenkt werden kann. Damit kann die Montage bzw. das Bereitstellen der Aufnahmevorrichtung vereinfacht werden und darüber hinaus auch eine längere Lagerdauer ohne nachteiligen Abbau des Unterdrucks erzielt werden. Darüber hinaus kann so ausgehend von der Bereitschaftsposition des Trennelements innerhalb des Aufnahmeraums und der nachfolgend einzunehmenden Trennstellung auch in dieser eine einwandfreie und dichtende Abtrennung der beiden in Axialrichtung beidseits des Trennelements angeordneten Teilräume des Aufnahmeraums sichergestellt werden. Damit kann auch in der Trennstellung eine einwandfreie und dauerhafte Abtrennung der beiden voneinander separierten Medien bzw. Phasen des Gemisches erfolgen.

Durch die Wahl der kugeligen Form des Trennelements wird ein Anhaften von Bestandteilen der zu trennenden Phasen aufgrund der sphärischen Oberfläche erschwert bzw. überhaupt verhindert. Durch das Anordnen des Trennelements im Bodenbereich des röhrchenförmigen Aufnahmebehälters kann so beispielsweise bei der Blutabnahme auf standardmäßige Abnahmevorrichtungen zurückgegriffen werden. Zum Einfüllen des Gemisches, insbesondere des Blutes, ist lediglich das Dichtelement der Verschlusseinheit zu durchstechen, wobei das Trennelement davon nicht betroffen ist. Damit kann das Einfüllen ungehindert in bekannter Weise durchgeführt werden. Weiters kann durch das Anordnen des Trennelements im Bodenbereich der weitere standardmäßige Herstellungsprozess der Aufnahmevorrichtung Anwendung finden, wie dieser bei den standardisierten Abnahme- bzw. Aufnahmevorrichtungen bei der Blutabnahme eingesetzt wird. Durch die Vorpositionierung und die damit verbundene klemmende Halterung des Trennelements in seiner Bereitschaftsposition kann während des Transports bis hin zum Einfüllvorgang eine definierte Positionsbeibehaltung des Trennelements innerhalb des Aufnahmeraums sichergestellt werden. Durch die Wahl des elastisch verformbaren Werkstoffs des Trennelements kann des Weiteren auf zusätzliche Bauteile innerhalb des Aufnahmebehälters verzichtet werden. Damit kann nicht nur eine sichere Bereitschaftsposition sondern auch nach Beendigung des Separiervorgangs eine einwandfreie, dauerhafte Trennung erreicht werden. Des Weiteren wird durch die vorpositionierte Bereitschaftsposition stets ein ausreichendes Einfüllvolumen sichergestellt, um eine ausreichende Probenmenge in den Aufnahmeraum einfüllen zu können.

Vorteilhaft ist auch eine weitere Ausführungsform nach Anspruch 2, da dadurch bereits in der Bereitschaftsposition in Abhängigkeit von den Abmessungsunterschieden auch bei Temperaturschwankungen sowie Stoßbelastungen stets eine relative Fixposition des Trennelements innerhalb des Aufnahmeraums sichergestellt werden kann.

Vorteilhaft ist weiters eine Ausbildung nach Anspruch 3, da dadurch für die Bildung des Strömungskanals zwischen dem Trennelement und der Seitenwand des Aufnahmebehälters ein ausreichendes Deformationsverhalten des Trennelements erreicht werden kann. Darüber hinaus wird durch die Wahl des Werkstoffs ein zusammengehöriges, einteiliges Bauteil geschaffen, von dem Abtrennungen von Materialpartikel nicht nur während des Zentriervorgangs vermieden werden können. Die Verunreinigung der eingefüllten Probe durch abgetrennte Partikel des Trennelements, wie dies beispielsweise bei der Verwendung eines Gels sehr leicht auftreten kann, kann damit verhindert werden.

Durch die Ausbildung nach Anspruch 4 ist es möglich, ein sicheres Aufschwimmen des Trennelements an der Trennebene bzw. Trennfläche zwischen den beiden zu separierenden Phasen zu gewährleisten.

Nach einer anderen Ausführungsvariante gemäß Anspruch 5 wird insbesondere bei Blut als zu separierendes Gemisch das Einbringen desselben in den Aufnahmeraum erleichtert. Darüber hinaus kann aber auch die Füllmenge und damit das Volumen der Probe je nach Ausmaß des gewählten Unterdruckes festgelegt werden.

Vorteilhaft ist auch eine Weiterbildung nach Anspruch 6, da dadurch für den in den Aufnahmeraum einzusetzenden Dichtstopfen im Bereich des offenen Endes ein besserer Dichtsitz erreicht werden kann.

Durch die Ausbildung nach Anspruch 7 können unterschiedlichste Füllmengen des Gemisches separiert werden. Des Weiteren kann so auch über eine größere Axialerstreckung stets eine sichere, dichtende Anlage des Trennelements in dessen Trennstellung erzielt werden.

Vorteilhaft ist auch eine Ausbildung nach Anspruch 8, da so ein leichteres Einsetzen des Trennelements in den Aufnahmeraums erfolgen kann. Dadurch wird erst im Bereich des zweiten Teilabschnitts die dichtende Anlage des Trennelements an der Innenfläche sichergestellt. Darüber hinaus kann so aber auch der Herstellvorgang, insbesondere der Entformvorgang bei einem Spritzgussprozess erleichtert werden.

Gemäß einer Ausbildung, wie im Anspruch 9 beschrieben, können so Ablagerungen von schwereren Bestandteilen des Gemisches während des Separiervorgangs vermieden werden. Damit kann eine nachträgliche Verunreinigung der leichteren Phase des Gemisches nach erfolgtem Zentrifugiervorgang ebenfalls vermieden werden.

Die Aufgabe der Erfindung kann aber eigenständig auch durch ein Vorgehen gemäß den Merkmalen des Anspruchs 10 gelöst werden. Die sich aus der Merkmalskombination dieses Anspruchs ergebenden Vorteile liegen darin, weil so das Trennelement durch einfache Vorverformung und Aufnahme innerhalb des Montagerohrs bis in den Bodenbereich des Aufnahmebehälters verbracht werden kann und erst dort in der vorbestimmten Bereitschaftsposition positioniert werden kann. Damit könnte sogar auf ein vorheriges Evakuieren des Aufnahmeraums verzichtet werden, da bei entsprechender Dimensionswahl des Montagerohrs und ausreichender Vorverformung des Trennelements dieses zuerst an der Bodenwand des Bodens beim Herausdrücken zur Anlage gebracht werden kann, bevor dieses vollständig aus dem Montagerohr herausgedrückt wird. Damit kann ein ungewollter Einschluss von Umgebungsluft zwischen dem Trennelement und dem Boden verhindert werden.

Die Aufgabe der Erfindung kann aber eigenständig auch durch ein Vorgehen gemäß den Merkmalen des Anspruchs 11 gelöst werden. Bei dieser vorteilhaften Vorgehensweise ist zwar ein Durchstechen bzw. Durchdringen des Trennelements vom Montagedorn notwendig, jedoch kann das Trennelement so im Bodenbereich des Aufnahmebehälters angeordnet werden. Damit kann ein Einschluss von Luft bzw. Restluftmengen gesichert verhindert werden.

Die Aufgabe der Erfindung kann aber eigenständig auch durch ein Vorgehen gemäß den Merkmalen des Anspruchs 12 gelöst werden. Vorteilhaft ist bei diesem Vorgehen, dass so ein Einschluss einer Restluftmenge zwischen dem Trennelement und dem Boden verhindert werden kann. Damit findet auch keine negative Beeinflussung der Lagerdauer statt, da bei einem evakuierten Aufnahmeraum kein zusätzlicher Abbau des darin aufgebauten Unterdruckes durch eingeschlossene Restluftmengen stattfinden kann.

Durch die Wahl der kugeligen Form des Trennelements wird ein Anhaften von Bestandteilen der zu trennenden Phasen aufgrund der sphärischen Oberfläche erschwert bzw. überhaupt verhindert. Durch das Anordnen des Trennelements im Bodenbereich des röhrchenförmigen Aufnahmebehälters kann so beispielsweise bei der Blutabnahme auf standardmäßige Abnahmevorrichtungen zurückgegriffen werden. Zum Einfüllen des Gemisches, insbesondere des Blutes, ist lediglich das Dichtelement der Verschlusseinheit zu durchstechen, wobei das Trennelement davon nicht betroffen ist. Damit kann das Einfüllen ungehindert in bekannter Weise durchgeführt werden. Weiters kann durch das Anordnen des Trennelements im Bodenbereich der weitere standardmäßige Herstellungsprozess der Aufnahmevorrichtung Anwendung finden, wie dieser bei den standardisierten Abnahme- bzw. Aufnahmevorrichtungen bei der Blutabnahme eingesetzt wird. Durch die Vorpositionierung und die damit verbundene klemmende Halterung des Trennelements in seiner Bereitschaftsposition kann während des Transports bis hin zum Einfüllvorgang eine definierte Positionsbeibehaltung des Trennelements innerhalb des Aufnahmeraums sichergestellt werden. Durch die Wahl des elastisch verformbaren Werkstoffs des Trennelements kann des Weiteren auf zusätzliche Bauteile innerhalb des Aufnahmebehälters verzichtet werden. Damit kann nicht nur eine sichere Bereitschaftsposition sondern auch nach Beendigung des Separiervorgangs eine einwandfreie, dauerhafte Trennung erreicht werden. Des Weiteren wird durch die vorpositionierte Bereitschaftsposition stets ein ausreichendes Einfüllvolumen sichergestellt, um eine ausreichende Probenmenge in den Aufnahmeraum einfüllen zu können.

Weiters ist ein Vorgehen gemäß den im Anspruch 13 angegebenen Merkmalen vorteilhaft, weil insbesondere bei Blut als zu separierendem Gemisch das Einbringen desselben in den Aufnahmeraum erleichtert werden kann. Darüber hinaus kann aber auch die Füllmenge und damit das Volumen der Probe je nach Ausmaß des gewählten Unterdruckes festgelegt werden.

Die Aufgabe der Erfindung kann aber unabhängig davon auch durch ein Verfahren zum Trennen eines Gemisches, insbesondere von Blut, gemäß den im Anspruch 14 angegebenen Merkmalen gelöst werden. Die sich aus der Merkmalskombination dieses Anspruchs ergebenden Vorteile liegen darin, dass so mit einer erfindungsgemäß ausgebildeten Aufnahmevorrichtung und dem darin angeordneten Trennelement ein standardisierter Zentrifugiervorgang durchgeführt werden kann. Nach Beendigung desselben wird aufgrund der hier stattfindenden ausschließlichen elastischen Verformung des Trennelements eine sichere dichtende Lage in dessen Trennstellung erreicht. Durch die Wahl der Kugelform in Verbindung mit dem elastischen Werkstoff kann so aber auch ein Abtrennen von Teilmengen des Trennelements vermieden werden, wie dies eine große Gefahr bei der Verwendung von Trenngels ist.

Schließlich ist eine weitere vorteilhafte Vorgehensweise im Anspruch 15 beschrieben, wodurch auch kleinste Bestandteile mit einer höheren Dichte sicher in den zwischen dem Trennelement und dem Boden des Aufnahmebehälters ausgebildeten Teilraum verbracht werden können. Dadurch wird eine sehr hohe Reinheit der leichteren und zwischen dem Trennelement und dem offenen Ende des Aufnahmebehälters verbrachten Phase erzielt.

Zum besseren Verständnis der Erfindung wird diese anhand der nachfolgenden Figuren näher erläutert.

Es zeigen jeweils in stark vereinfachter, schematischer Darstellung:
- Fig. 1: eine Aufnahmevorrichtung mit darin eingefülltem Gemisch, vor dem Beginn des Separiervorgangs, im Axialschnitt;
- Fig. 2: die Aufnahmevorrichtung nach Fig. 1, nach Beendigung des Separiervorgangs;
- Fig. 3: eine erste Montagemöglichkeit zur Einbringung des Trennelements in den Aufnahmeraum des Aufnahmebehälters;
- Fig. 4: eine zweite Möglichkeit zur Einbringung des Trennelements in den Aufnahmeraum des Aufnahmebehälters;
- Fig. 5: eine dritte Montagemöglichkeit zur Einbringung des Trennelements in den Aufnahmeraum des Aufnahmebehälters.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, un-ten, seitlich usw. auf die unmittelbar beschriebene sowie dargestellte Figur bezogen und sind diese Lageangaben bei einer Lageänderung sinngemäß auf die neue Lage zu übertragen.

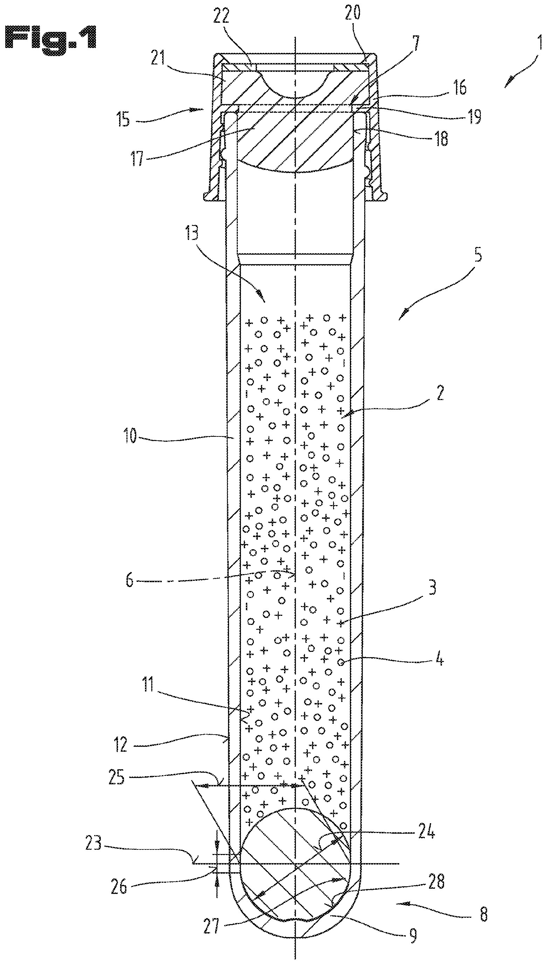

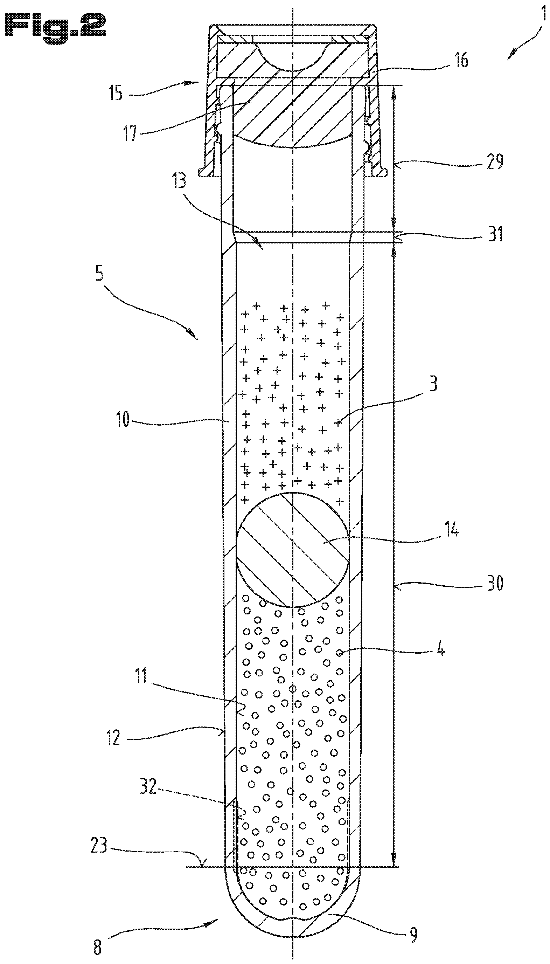

In den Fig. 1 und 2 ist eine Aufnahmevorrichtung 1 für ein Gemisch 2 bzw. Substanzen aus zumindest zwei zueinander unterschiedlichen Bestanteilen bzw. Medien, wie beispielsweise Körperflüssigkeiten, Gewebeteile bzw. die Gewebekulturen gezeigt, welche dazu ausgebildet ist, das in der Aufnahmevorrichtung 1 befindliche Gemisch 2 in zumindest zwei seiner Bestandteile separieren zu können. Dies erfolgt zumeist durch Beaufschlagung mit einer Zentrifugalkraft auf mechanische Weise. Das Gemisch 2 zum Trennen bzw. Separieren liegt dabei bevorzugt in einem flüssigen Aggregatzustand vor.

Bevorzugt wird diese Aufnahmevorrichtung 1 zum Trennen von Blut eingesetzt, wobei ein Bestandteil desselben hier als leichtere Phase 3 mit einer geringeren Dichte und eine schwerere Phase 4 mit einer dazu größeren Dichte bezeichnet wird. Bei Blut ist beispielsweise die leichtere Phase 3 das Serum oder das Plasma, wobei die schwere Phase zelluläre Bestandteil, wie beispielsweise Erythrozyten, Leukozyten und Thrombozyten, sind.

Dieses Separieren bzw. Trennen des Gemisches 2 in seine beiden Phasen 3, 4 kann beispielsweise physikalisch durch Zentrifugierung auf herkömmliche Art und Weise erfolgen. So kann ausgehend von der Ruheposition bis zum Erreichen einer radialen Zentrifugalbeschleunigung von 1.000 g bis 5.000 g, vorzugsweise zwischen 1.800 g und 3.500 g durchgeführt werden, wobei "g" die Erdbeschleunigung ist und der Wert von 1 g = 9,81 m/s² beträgt. Dadurch ist es möglich, die leichtere Phase 3 von der schwereren, zumeist festeren Phase 4 abzuscheiden bzw. nach den unterschiedlichen Dichtewerten zu trennen, wie dies in den nachfolgenden Figuren noch detailliert beschrieben wird.

Die Aufnahmevorrichtung 1 umfasst einen in etwa zylinderförmig ausgebildeten zumeist rohrförmigen Aufnahmebehälter 5, der in Richtung einer Längsachse 6 voneinander distanzierte erste und zweite Enden 7, 8 aufweist. So ist bei diesem Ausführungsbeispiel das erste Ende 7 offen und das zweite Ende 8 mit einem Boden 9 verschlossen. Weiters umfasst der Aufnahmebehälter 5 eine Seitenwand 10 bzw. Behälterwand, welche sich zwischen dem ersten und dem zweiten Ende 7, 8 erstreckt und ihrerseits eine Innenfläche 11 sowie eine Außenfläche 12 aufweist. Die Seitenwand 10 und der Boden 9 umgrenzen weiters einen Aufnahmeraum 13, in welchen das zu trennende Gemisch 2 einzufüllen bzw. einzubringen ist.

Die Aufnahmevorrichtung 1 umfasst weiters ein Trennelement 14, welches innerhalb bzw. im Aufnahmeraum 13 angeordnet ist und bevorzugt vollständig bzw. durchgängig aus einem elastisch verformbaren Werkstoff gebildet ist. Weiters kann der dabei gewählte Werkstoff zur Bildung des Trennelements 14 nach einem Durchstichvorgang selbstverschließend ausgebildet sein. Dies bedeutet, dass sich der Werkstoff nach Entfernen des Durchstichelements im Bereich der Trennstelle bzw. Durchstichstelle selbsttätig elastisch soweit rückstellt, dass das Trennelement 14 gasdicht und/oder flüssigkeitsdicht ist. Als elastisch verformbare Werkstoffe können beispielsweise thermoplastische Elastomere (TPE), Silikon, Gummi, Pharmagummi, Silikonkautschuk oder dergleichen eingesetzt werden.

Weiters kann die Aufnahmevorrichtung 1 auch noch eine abnehmbare Verschlusseinheit 15 umfassen, welche im vorliegenden Fall das erste, offene Ende 7 des Aufnahmebehälters 5 verschließt.

Die Verschlusseinheit 15 kann ihrerseits wiederum unterschiedlichst ausgebildet sein, wobei diese im hier gezeigten Ausführungsbeispiel eine die offene Stirnseite des ersten Endes 7 umfassende Kappe 16 sowie ein darin gehaltenes Dichtelement 17, wie beispielsweise einen Dichtstopfen umfasst. Das Dichtelements 17 ist zumeist aus einem durchstechbaren, hochelastischen und selbstverschließenden Werkstoff, wie z.B. Pharmagummi, Silikonkautschuk oder Brombutylkautschuk, gebildet. Jener Teil des Dichtelements 17, welcher in das erste offene Ende 7 eingesetzt ist, weist in Axialrichtung gesehen eine Dichtfläche 18 auf, welche dichtend anliegend an der Innenfläche 11 ausgebildet ist. Damit kann der Aufnahmebehälter 5 in bekannter Weise verschlossen werden.

Die Kappe 16, insbesondere deren Kappenmantel ist konzentrisch zur Längsachse 6 angeordnet und kreisringförmig bzw. annähernd rohrförmig ausgebildet. Zwischen der Kappe 16 und dem Dichtelement 17 können Mittel zum Kuppeln, wie beispielsweise Kupplungsteile einer Kupplungsvorrichtung, vorgesehen sein. Diese Kupplungsteile können beispielsweise bei der Kappe 16 durch über deren Innenumfang vorragende, auch bereichsweise angeordnete Fortsätze 19, 20 gebildet sein. Das Dichtelement 17 kann weiters einen die Dichtfläche 18 radial überragenden Ansatz 21 aufweisen, welcher in den zwischen den Fortsätzen 19, 20 definierten Aufnahmeraum der Kappe 16 hineinragt. Zur leichteren Montage und besseren Fixierung des Ansatzes 21 zwischen den beiden Fortsätzen 19, 20 kann zwischen dem Ansatz 21 und dem hier auf der vom Aufnahmebehälter 5 abgewendeten Seite der Kappe 16 vorgesehenen Fortsatz 20 ein zusätzlicher Haltering 22 angeordnet bzw. vorgesehen sein. Der hier kreisringförmig ausgebildete Haltering 22 weist im Bereich der Längsachse 6 eine Durchgangsöffnung auf, welche den Zugang zum Dichtelement 17 ermöglicht. In bekannter Weise kann für die Befüllung des Aufnahmeraums 13 des Aufnahmebehälters 5 das Dichtelement 17 mit einer nicht näher dargestellten Kanüle durchstochen werden und auf diesem Wege eine Strömungsverbindung zwischen der Kanüle und dem Aufnahmeraum 13 geschaffen werden. Dies ist hinlänglich bekannt und wird deshalb auf eine detaillierte Beschreibung verzichtet.

Durch das Vorsehen bzw. die Anordnung des Fortsatzes 19 zwischen dem die Dichtfläche 18 überragenden Ansatz 21 und der offenen Stirnseite des Aufnahmebehälters 5 kann eine Verklebung bzw. eine starke Anhaftung des Ansatzes 21 direkt an der Stirnseite des Aufnahmebehälters 5 vermieden werden.

Das Trennelement 14 weist in seiner unverformten Ausgangsform eine überwiegend kugelig ausgebildete Raumform auf. Unter überwiegend kugelig wird hier verstanden, dass die grundsätzliche Raumform einer Kugel entspricht, wobei fertigungs- bzw. herstellungsbedingte Abweichungen von der Kugelform im Rahmen der Herstellgenauigkeit unter Einhaltung üblicher Toleranzabweichungen liegt.

Bei dieser Aufnahmevorrichtung 1 wird das Trennelement 14 vor dem Befüllen des Aufnahmeraums 13 mit dem zu trennenden Gemisch 2 im Bereich des mit dem Boden 9 verschlossenen, zweiten Endes 8 angeordnet. Wie aus der Darstellung der Fig. 1 zu ersehen ist, liegt das Trennelement 14 zumindest in einer senkrecht bezüglich der Längsachse 6 ausgerichteten Anlageebene 23 an der Innenfläche 11 der Seitenwand 10 an. Des Weiteren ist ein Außendurchmesser 24 des Trennelements 14 in der unverformten Ausgangsform des Trennelements 14 größer als eine Querschnittsabmessung 25 des Aufnahmeraums 13 in der Anlageebene 23. Aufgrund dieser Abmessungsunterschiede ist das Trennelement 14 an der Innenfläche 11 der Seitenwand 10 in einer vorgespannten Bereitschaftsposition positioniert daran gehalten. Je nach gewähltem Abmessungsunterschied kann ein Anlagebereich 26 des Trennelements 14 ausgehend von einer linienförmigen Berührung bzw. Anlage bis hin zu einem sich beidseits der Anlageebene 23 erstreckenden in etwa zylinderförmigen Anlagebereich 26 erstrecken.

So kann beispielsweise der Außendurchmesser 24 des Trennelements 14 in seiner unverformten Ausgangsform in einem Wertebereich zwischen 1 % und 10 %, insbesondere zwischen 2 % und 5 % größer ausgebildet sein, als die Querschnittsabmessung 25 des Aufnahmeraums 13 in der Anlageebene 23 bzw. im Bereich der Anlageebene 23. Das elastisch verformbar ausgebildete Trennelement 14 kann eine Shore A Härte aufweisen, welche ausgewählt ist aus einem Wertebereich zwischen 7 Shore A und 20 Shore A, insbesondere zwischen 9 Shore A und 12 Shore A. Besonders bevorzugt kann die Shore Härte einen Wert von 10 Shore A aufweisen.

Wird beispielsweise Blut als zu trennendes Gemisch 2 in die Aufnahmevorrichtung 1 eingefüllt, weisen die beiden Phasen 3, 4 zueinander eine unterschiedliche Dichte bzw. Dichtewerte auf. So weist bekanntlich die leichtere Phase 3 eine Dichte zwischen 1,02 kg/m³ und 1,03 kg/m³ und die schwerere Phase 4 eine Dichte zwischen 1,05 kg/m³ und 1,09 kg/m³ auf. Die Dichtewerte der beiden Phasen 3, 4 sind grundsätzlich zwischen Männern und Frauen gleich, jedoch das Mengenverhältnis zueinander unterschiedlich. Die Dichte von Vollblut unterliegt zumeist einer Schwankung, da die Anteile der beiden Phasen nicht immer gleich sind. Dies ist auch vom Geschlecht abhängig, wobei z.B. bei Männern die leichtere Phase 3 einen Volumenanteil im Vollblut in einem Wertebereich zwischen 50% und 60% aufweist. Bei Frauen liegt der Volumenanteil am Vollblut in einem Wertebereich zwischen 55% und 70%. Daraus resultiert auch ein Höhenunterschied der Lage der Trennfläche zwischen den beiden Phasen 3, 4, der somit nicht nur von der Gesamtfüllmenge, sondern auch vom Geschlecht des Patienten bzw. Spenders abhängig ist.

Um ein Aufschwimmen bzw. Anordnen des Trennelements 14 im Zuge des Trennvorgangs bzw. Separierens des Gemisches 2 zwischen den zu trennenden Phasen 3, 4 zu erzielen, soll das Trennelement 14 eine Dichte ausgewählt aus einem Wertebereich zwischen 1,02 kg/m³ und 1,09 kg/m³, insbesondere zwischen 1,03 kg/m³ und 1,04 kg/m³ aufweisen. Damit liegt der Dichtewert des Trennelements 14 bzw. dessen Werkstoff zwischen den Dichtewerten der leichteren Phase 3 und der schwereren Phase 4.

Um das Einfüllen bzw. Einbringen des Gemisches 2 in den Aufnahmeraum 13 bei verschlossenem Aufnahmebehälter 5 zu erleichtern, kann in bekannter Weise der verschlossene Aufnahmeraum 13 des Aufnahmebehälters 5 auf einen bezüglich des Umgebungsdruckes dazu geringeren Druck abgesenkt sein.

Die Raumform des Aufnahmebehälters 5 sowie der Verschlusseinheit 15, insbesondere der Kappe 16, im Bereich von deren Außenseiten bzw. Außenflächen wird bevorzugt so gewählt, dass diese den üblichen Standardabmessungen bzw. Raumformen entspricht. Damit kann eine standardisierte, nachfolgende Probenanalyse oder aber auch eine automatisierte Abnahme von Teilmengen der voneinander getrennten Phasen 3, 4 aus dem Aufnahmeraum 13 erfolgen. Die Innenfläche 11 der Seitenwand 10 geht in einem Übergangsbereich 27 in eine durch den Boden 9 gebildete Bodenfläche 28 über. Der Boden 9 weist bevorzugt eine kugelkalottenförmige Raumform auf.

Weiters ist bei diesem Ausführungsbeispiel gezeigt, dass die Innenfläche 11 der Seitenwand 10 in Axialrichtung gesehen mehrere unterschiedliche Teilabschnitte 29, 30 aufweisen kann. Das elastisch verformbar ausgebildete Trennelement 14 wird weiters bevorzugt eng anliegend an der durch den Boden 9 gebildeten Bodenfläche 28 angeordnet bzw. spaltfrei an die durch den Boden 9 gebildete Bodenfläche 28 angelegt. Dies ist in der Fig. 1 vereinfacht angedeutet.

Wie nun besser aus der Fig. 2 zu ersehen ist, ist ein dem ersten, offenen Ende 7 unmittelbar benachbarter erster Teilabschnitt 29 der Innenfläche 11 nahezu zylindrisch bezüglich der Längsachse 6 ausgebildet. Dabei kann eine Axiallänge des ersten Teilabschnitts 29 zumindest einer Längserstreckung der Dichtfläche 18 des in den Aufnahmeraum 13 eingesetzten Dichtelements 17 der Verschlusseinheit 15 entsprechen. Diese axiale Längserstreckung des ersten Teilabschnitts 29 kann aber auch dazu größer, wie beispielsweise dem doppelten oder dreifachen der Längserstreckung der Dichtfläche 18 gewählt sein.

Ein zweiter Teilabschnitt 30 der Innenfläche 11 kann ebenfalls nahezu zylindrisch bwzüglich der Längsachse 6 ausgebildet sein. So kann sich der zweite Teilabschnitt 30 kann ausgehend vom Übergangsbereich 27 der Innenfläche 11 zum Boden 9 hin in Richtung auf das erste offene Ende 7 über eine Axiallänge erstrecken, welche zumindest 50 %, bevorzugt 60 %, des Füllvolumens des Aufnahmeraums 13 entspricht. Es wäre aber auch möglich, dass sich der zweite Teilabschnitt 30 ausgehend vom Übergangsbereich 27 der Innenfläche 11 zum Boden 9 durchgängig bis hin zum ersten Teilabschnitt 29 im Bereich des ersten offenen Endes 7 erstreckt. Weiters ist hier noch zu ersehen, dass der erste Teilabschnitt 29 einen Durchmesser aufweist, welcher bezüglich des zweiten Teilabschnitts 30 dazu größer ausgebildet ist. Unter nahezu zylindrisch wird auch noch eine sehr geringe Konizität verstanden, welche dazu dient, eine einwandfreie Entformung des Aufnahmebehälters 5 bei Herstellung in einem Spritzgussprozess zu gewährleisten.

Um einen Übergang zwischen den die unterschiedlichen Durchmesser aufweisenden Teilabschnitten 29, 30 zu erreichen, kann anschließend an den ersten Teilabschnitt 29 auf seiner dem Boden 9 zugewendeten Seite ein kegelförmig verjüngend ausgebildeter Übergangsabschnitt 31 ausgebildet bzw. vorgesehen sein.

Wie weiters in der Fig. 2 im Bereich des verschlossenen zweiten Endes 8 in strichlierten Linien angedeutet, kann zwischen dem Boden 9 und dem zweiten Teilabschnitt 30 der Innenfläche 11 zumindest ein Kanal 32 angeordnet oder ausgebildet sein. Dieser Kanal 32 bzw. die Kanäle können beispielsweise entweder durch Vertiefungen in der Innenfläche 11 der Seitenwand 10 und/oder aber auch Stege oder Vorsprünge, die in radialer Richtung über die Innenfläche 11 in Richtung auf die Längsachse 6 vorragen, ausgebildet sein. Der oder die Kanäle 32 dienen dazu, wenn das im Aufnahmeraum 13 angeordnete Trennelement 14 sich in der Bereitschaftsposition im Bereich des Bodens 9 befindet, jene beidseits der Anlageebene 23 befindlichen Teilräume des Aufnahmeraums 13 miteinander strömungstechnisch zu verbinden. Dies kann dazu dienen, wenn der Aufnahmeraum 13 auf einen gegenüber dem Umgebungsdruck dazu geringeren Druck abgesenkt wird, dass auch jener Teilraum, der sich zwischen der Anlageebene 23 und dem Boden 9 befindet, ebenfalls auf einen geringeren Druck abgesenkt werden kann. Dies könnte dann der Fall sein, wenn das Trennelement 14 ohne vorheriges Evakuieren in den Aufnahmeraum 13 eingebracht wird und dabei die im Aufnahmeraum 13 enthaltene Luft bzw. eine Teilmenge derselben in dem dem Boden 9 benachbarten Teilraum eingeschlossen wird.

Um das Trennelement 14 in seine im Bodenbereich befindliche Bereitschaftsposition vor dem Verschließen des Aufnahmeraums durch die Verschlusseinheit 15 sowie noch vor dem Befüllen des Aufnahmeraums mit dem Gemisch 2 zu verbringen, können dazu unterschiedliche Bereitstellungsverfahren bzw. Vorgänge zur Bildung der Aufnahmevorrichtung 1 gewählt werden.

Der Aufnahmebehälter 5 kann in bekannter Weise beispielsweise in einem Spritzgussprozess aus einem Kunststoffmaterial hergestellt werden, wobei dieser in Richtung der Längsachse 6 die voneinander distanzierten ersten und zweiten Enden 7 aufweist. Durch den Boden 9 und die Seitenwand 10 wird der Aufnahmeraum 13 umgrenzt. Der Aufnahmebehälter 5 ist zumindest im Bereich der Seitenwand 10 alleinig und ausschließlich durch das Kunststoffmaterial gebildet, welches auch die Innenfläche des Aufnahmebehälters 5 bildet. Damit bildet die Seitenwand 10 mit ihrer Innenfläche eine stabile und feste Anlagefläche für das Trennelement 14 aus. Eine mögliche Beschichtung mit einem Wirkstoff kann noch auf der Innenfläche der Seitenwand 10 sowie gegebenenfalls des Bodens 9 aufgebracht sein. Das Trennelement 14 wird vollständig bzw. durchgängig aus dem zuvor beschriebenen, elastisch verformbaren Werkstoff gebildet und anschließend im Aufnahmeraum 13 angeordnet. Unter vollständig bzw. durchgängig wird hier verstanden, dass das Trennelement 14 alleinig und somit gänzlich aus einem einzigen Werkstoff besteht. Hierzu werden nachfolgend die unterschiedlichsten Montagemöglichkeiten bzw. Verfahren dazu angegeben.

Wesentlich dabei ist, dass das Trennelement 14 mit einer überwiegend kugeligen Raumform ausgebildet wird. Des Weiteren wird das Trennelement 14 noch vor dem Befüllen des Aufnahmeraums 13 mit dem zu trennenden Gemisch 2 zu dem im Bereich des mit dem Boden 9 verschlossenen zweiten Endes 8 verbracht und dort aufgrund der zuvor beschriebenen Abmessungsunterschiede in der vorgespannten Bereitschaftsposition positioniert gehalten. Dabei liegt die Außenfläche des Trennelements 14 im Bereich der Anlageebene 23 bzw. dem in Axialrichtung länger ausgebildeten Anlagebereich 26 an der Innenfläche 11 der Seitenwand 10 an.

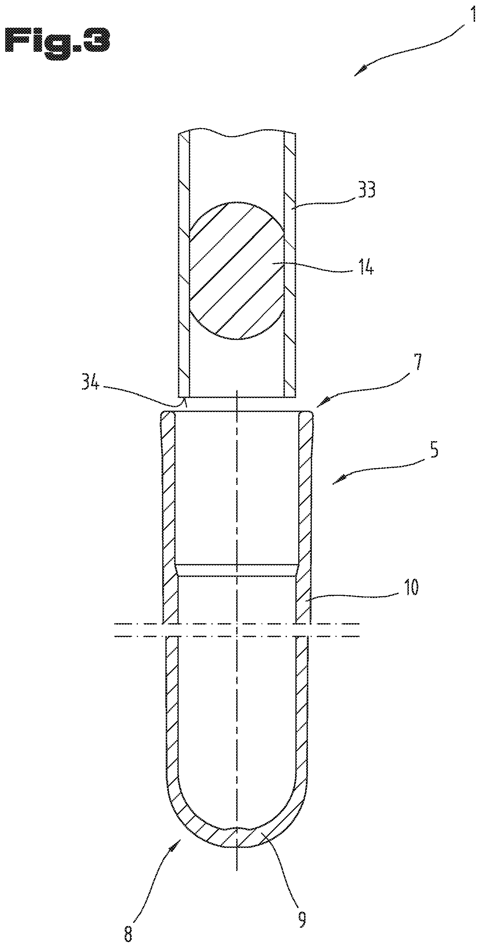

In der Fig. 3 ist eine erste Montagemöglichkeit bzw. ein Verfahren zum Bereitstellen mit dem Einsetzen des Trennelements 14 in den Bereich des Bodens 9 des Aufnahmebehälters 5 vereinfacht dargestellt. Dieses Vorgehen kann eine für sich eigenständige Lösung darstellen, wobei wiederum für gleiche Teile gleiche Bezugszeichen bzw. Bauteilbezeichnungen wie in den vorangegangenen Fig. 1 und 2 verwendet werden. Um unnötige Wiederholungen zu vermeiden, wird auf die detaillierte Beschreibung in den vorangegangenen Fig. 1 und 2 hingewiesen bzw. Bezug genommen.

So wird hier das unverformte kugelige Trennelement 14 vor dem Einbringen in den Aufnahmeraum 13 in ein eigenes, vereinfacht angedeutetes Montagerohr 33 eingesetzt. Dabei weist das Montagerohr 33 eine bezüglich einer lichten Innenabmessung des Aufnahmeraums 13 dazu geringere Außenabmessung auf, um ungehindert in den Aufnahmeraum 13 eingeschoben werden zu können. Ist das Trennelement 14 durch elastische Verformung in das Montagerohr 33 eingesetzt bzw. eingebracht, wird das Montagerohr 33 soweit in den Aufnahmeraum 13 hineinverstellt bzw. verbracht, sodass dessen Stirnende 34 dem Boden 9 benachbart angeordnet ist. Wurde diese Montageposition erreicht, kann anschließend das Trennelement 14 aus dem Montagerohr 33 herausgedrückt werden.

Aufgrund von gewählten Abmessungsunterschieden bzw. Durchmesserdifferenzen zwischen dem Montagerohr 33 und der Seitenwand 10 des Aufnahmebehälters 5 kann beispielsweise beim Herausdrücken des Trennelements 14 aus dem Montagerohr 33 auch noch die zwischen dem Boden 9 und dem Trennelement 14 angeordnete Luftmenge entweichen. Die erfolgt beispielsweise zwischen der Innenfläche 11 der Seitenwand 10 und der Außenfläche des Montagerohrs 33 aus dem Aufnahmeraum 13. Damit kann verhindert werden, dass in dem zwischen der Anlageebene 23 und dem Boden 9 ausgebildeten Teilraum des Aufnahmeraums 13 keine Umgebungsluft eingeschlossen wird.

Weiters könnten aber auch mehrere Trennelemente 14 gleichzeitig innerhalb des Montagerohrs 33 hintereinander angeordnet werden, um so nacheinander in mehreren Aufnahmebehältern 5 jeweils eines der Trennelemente 14 einzubringen. Damit könnten weiters Montagezeiten reduziert werden, da nicht nach jedem einzelnen Positioniervorgang ein neues, weiteres Trennelement 14 in das Montagerohr 33 einzusetzen ist.

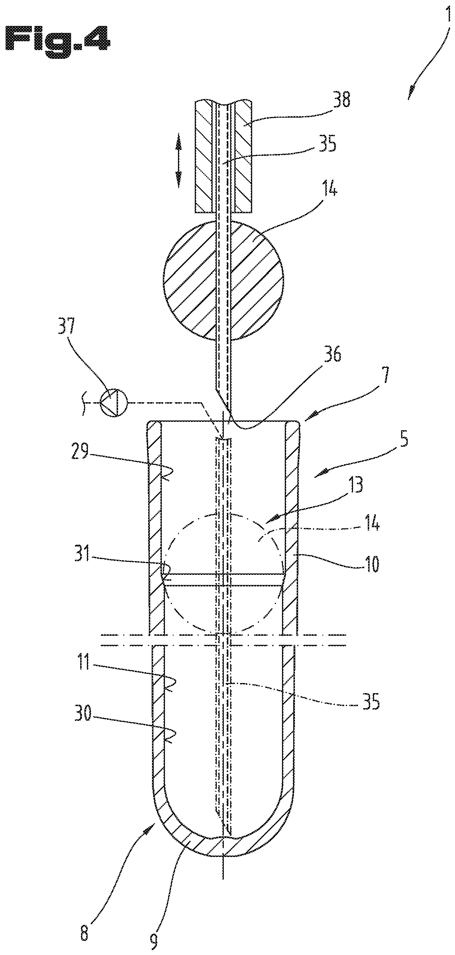

In der Fig. 4 ist eine zweite Montagemöglichkeit bzw. ein Verfahren zum Einsetzen des Trennelements 14 in den Bereich des Bodens 9 des Aufnahmebehälters 5 vereinfacht dargestellt. Auch dieses Vorgehen kann eine für sich eigenständige Lösung darstellen, wobei wiederum für gleiche Teile gleiche Bezugszeichen bzw. Bauteilbezeichnungen wie in den vorangegangenen Fig. 1 bis 3 verwendet werden. Um unnötige Wiederholungen zu vermeiden, wird auf die detaillierte Beschreibung in den vorangegangenen Fig. 1 bis 3 hingewiesen bzw. Bezug genommen.

Bei diesem hier vereinfacht dargestellten Montagevorgang wird das Trennelement 14 von einem hohl ausgebildeten Montagedorn 35 durchstochen, wobei der Montagedorn 35 das Trennelement 14 vollständig durchdringt. Das Durchstechen des elastischen Werkstoffes des Trennelements 14 ist jedoch derart auszuführen, dass der Werkstoff des Trennelements 14 lediglich im Bereich des Montagedorns 35 durchtrennt wird, jedoch kein Volumenanteil des Trennelements 14 davon abgetrennt wird. Dabei kann von einem Aufspießen des Trennelements 14 an dem hohl ausgebildeten Montagedorn 35 gesprochen werden. Ist dies erfolgt, wird dann das Trennelement 14 mitsamt dem Montagedorn 35 bzw. überhaupt mittels des Montagedorns 35 zum offenen Ende 7 verbracht und an der Innenfläche 11 der Seitenwand 10 dichtend angelegt. Anschließend wird der Montagedorn 35 mit seinem dem Boden 9 zugewendeten Stirnende 36 soweit in den Aufnahmeraum 13 eingeschoben, dass das Stirnende 36 dem Boden 9 benachbart angeordnet ist. Durch die zuvor beschriebenen Abmessungsunterschiede zwischen dem Trennelement 14 und den beiden Teilabschnitten 29, 30 kann z.B. das Trennelement 14 im Bereich des Übergangsabschnitts 31 bzw. in dem an den ersten Teilabschnitt 29 in Richtung auf den Boden 9 unmittelbar anschließenden Teilabschnitt 30 der Innenfläche 11 vorpositioniert gehalten werden. Dabei ist auf eine rundum dichtende Anlage des Trennelements 14 zu achten.

Wird der zweite Teilabschnitt 30 ausgehend vom Übergangsbereich 27 bzw. der Anlageebene 23 nur über einen Teilabschnitt der Axialerstreckung bis hin zum Übergangsabschnitt 31 nahezu zylindrisch ausgebildet, kann das Trennelement 14 noch weiter in den Aufnahmeraum 13 in Richtung auf den Boden 9 eingeschoben werden. Liegt das Trennelement 14 rundum dichtend an der Innenfläche 11 der Seitenwand 10 an, kann der zwischen dem Boden 9 und dem Trennelement 14 abgedichtete Teilraum des Aufnahmeraums 13 über den das Trennelement 14 durchragenden, hohlen Montagedorn 35 auf einen bezüglich des äußeren Umgebungsdruckes dazu geringeren Druck abgesenkt werden. Dazu ist vereinfacht eine Unterdruckeinheit 37 angedeutet.

Durch das Aufbauen einer Druckdifferenz zwischen dem durch das Trennelement 14 abgedichteten Teilraum des Aufnahmeraums 13 und der äußeren Umgebung, wird das Trennelement 14 entlang des Montagedorns 35 gleitend zu dem zweiten, durch den Boden 9 verschlossenen Ende 8 des Aufnahmebehälters 5 verlagert.

Ist die zuvor beschriebene Bereitschaftsposition des Trennelements 14 im Aufnahmeraum 13 erreicht, kann der Montagedorn 35 aus dem Trennelement 14 herausgezogen werden. Beim Herausverstellen des Montagedorns 35 aus dem Trennelement 14 kann noch ein Positionierelement 38hilfreich sein, welches z.B. ebenfalls rohrförmig ausgebildet ist und das Trennelement 14 solange in der Bereitschaftsposition hält, bis dass der Montagedorn 35 aus dem Trennelement 14 herausgezogen ist. Anschließend können das Montagerohr 35 und das Positionierelement 38 gemeinsam aus dem Aufnahmebehälter 5 herausverstellt werden.

Anschließend daran kann, falls dies gewünscht wird, vor dem Verschließen des offenen ersten Endes 7 mit der Verschlusseinheit 15 der Aufnahmeraum 13 auf einen gegenüber dem äußeren Umgebungsdruck dazu geringeren Druck abgesenkt werden. Dies ist hinlänglich bekannt und dient zur leichteren und sichereren Befüllung des Aufnahmeraums 13 mit dem darin aufzunehmendem Gemisch 2. Dies ist insbesondere bei der Abnahme von Blut von Vorteil.

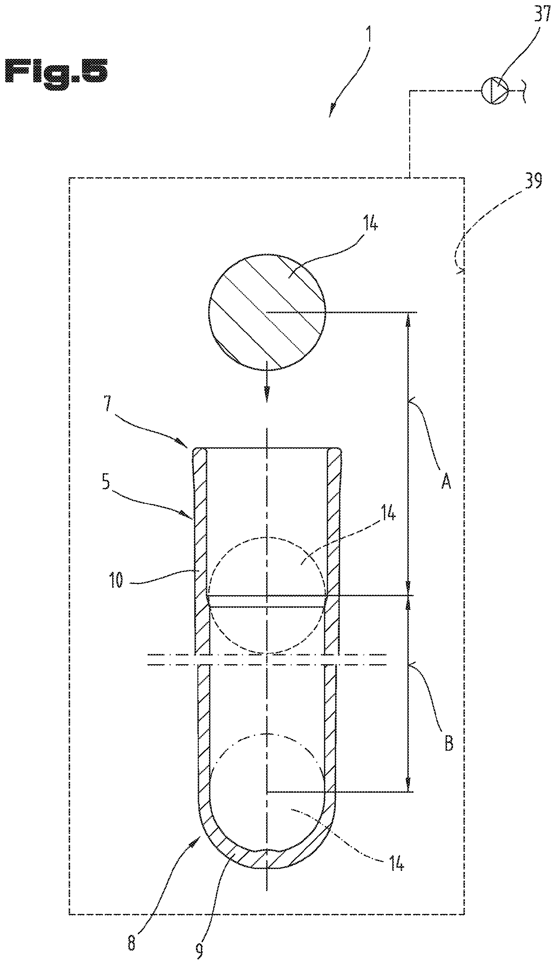

In der Fig. 5 ist eine dritte Montagemöglichkeit bzw. ein Verfahren zum Einsetzen des Trennelements 14 in den Bereich des Bodens 9 des Aufnahmebehälters 5 vereinfacht dargestellt. Auch dieses Vorgehen kann eine für sich eigenständige Lösungssmöglichkeit darstellen, wobei wiederum für gleiche Teile gleiche Bezugszeichen bzw. Bauteilbezeichnungen wie in den vorangegangenen Fig. 1 bis 4 verwendet werden. Um unnötige Wiederholungen zu vermeiden, wird auf die detaillierte Beschreibung in den vorangegangenen Fig. 1 bis 4 hingewiesen bzw. Bezug genommen.

Bei diesem hier beschriebenen Bereitstellungsverfahren bzw. Verfahren zum Einsetzen des Trennelements 14 in den Aufnahmeraum 13 des Aufnahmebehälters 5 wird vor dem Verschließen des Aufnahmebehälters 5 mit der Verschlusseinheit 15 der den Aufnahmebehälter 5 umgebende Bereich auf einen bezüglich des äußeren Umgebungsdruckes dazu geringeren Druck abgesenkt. Dies kann beispielsweise ein einer in strichlierten Linien angedeuteten Kammer 39 erfolgen. Das Absenken des Druckes innerhalb der Kammer 39 kann beispielsweise durch eine schematisch vereinfacht dargestellte Unterdruckeinheit 37 erfolgen.

Durch das Absenken des Druckes innerhalb der Kammer 39 herrscht in dieser ein gegenüber der äußeren Umgebung dazu reduzierter Druck. Da das erste offene Ende 7 des Aufnahmebehälters 5 noch nicht mit der Verschlusseinheit 15 verschlossen ist, herrscht auch dieser reduzierte Druck im Aufnahmeraum 13. Im Zustand des abgesenkten Druckes wird dann das Trennelement 14 gemäß eingetragenem Verstellweg "A" in das erste offene Ende 7 des Aufnahmebehälters 5 verbracht und dort soweit in Richtung auf den Boden 9 verlagert, dass das Trennelement 14 an der Innenfläche 11 bevorzugt dichtend zur Anlage kommt bzw. gebracht wird. Diese Zwischenstellung des Trennelements 14 ist in strichlierten Linien dargestellt.

Nachfolgend wird der Aufnahmebehälter 5 zumindest dem äußeren Umgebungsdruck ausgesetzt, wobei durch den dabei aufgebauten Druckunterschied zwischen dem Aufnahmeraum 13 und der äußeren Umgebung das Trennelement 14 gemäß dem weiteren eingetragenen Verstellweg "B" zu dem zweiten, durch den Boden 9 verschlossenen Ende 8 verlagert wird. Die dabei erreichte Bereitschaftsposition ist in strich-punktierten Linien angedeutet.

Das Aussetzen des Aufnahmebehälters 5 mit dem vorpositioniert darin angeordneten Trennelement 14 kann beispielsweise auch innerhalb der Kammer 39 erfolgen. So kann es beispielsweise möglich sein, die Druckdifferenz nur durch den äußeren Umgebungsdruck auszubilden. Es wäre aber auch möglich, innerhalb der Kammer 39 einen bezüglich des Umgebungsdruckes dazu höheren Druck aufzubauen und damit die Druckdifferenz zwischen dem im Aufnahmeraum 13 reduzierten Druck und jenem in der Kammer 39 herrschenden Druck zu erhöhen. Dabei kann auch von einem Überdruck gesprochen werden. Aufgrund des dabei aufgebauten Druckunterschiedes zwischen dem durch das Trennelement 14 abgeschlossenen Aufnahmeraum 13 und dem das Trennelement 14 umgebenden Raum wird dieses zu dem zweiten, durch den Boden 9 verschlossenen Ende 8 verlagert.

Da vor dem dichtenden Einsetzen des Trennelements 14 in den evakuierten Aufnahmeraum 13 somit nahezu keine bzw. keine Restluftmenge mehr darin enthalten ist, besteht auch nicht die Gefahr, dass Restluftmengen in dem zwischen der Anlageebene 23 bzw. dem Anlagebereich 26, der Bodenfläche 28 sowie dem Trennelement 14 ausgebildeten Teilraum bzw. Teilabschnitt des Aufnahmeraums 13 eingeschlossen werden kann.

Befindet sich das Trennelemente 14 in seiner Bereitschaftsposition im Bereich des Bodens 9 kann wiederum der Aufnahmeraum 13 des Aufnahmebehälters 5 auf einen bezüglich des Umgebungsdruckes dazu geringeren Druck abgesenkt werden und anschließend die Verschlusseinheit 15 auf den Aufnahmebehälter 5 im Bereich von dessen offenen Endes 7 aufgesetzt und damit verschlossen werden.

Dabei sei erwähnt, dass der Aufnahmebehälter 5 selbst aus den unterschiedlichsten Werkstoffen gebildet werden kann. Bevorzugt werden dabei Kunststoffe eingesetzt, wobei aber auch der Werkstoff Glas Verwendung finden kann. So kann beispielsweise der Kunststoff aus der Gruppe von Polyethylenterephthalat (PET), Polypropylen (PP), Polyethylen (PE), Polystyrol (PS), High-Density-Polyethylen (PE-HD), Acrylnitril-Butadien-Styrol-Copolymere (ABS), ultrahochmolekulares Polyethylen mit sehr hoher molarer Masse (PE-UHMW), Polycarbonat (PC), Polyamid (PA), Polyoxymethylen (POM) gewählt ist.

Darüber hinaus ist es möglich, entweder noch vor dem Einbringen bzw. Einsetzen des Trennelements 14 in den Aufnahmeraum 13 die Innenfläche 11 der Seitenwand 10 mit einer Beschichtung zu versehen. Diese Beschichtung kann beispielsweise dazu dienen bzw. ausgebildet sein, das in den Aufnahmeraum 13 aufzunehmende Gemisch 2 zu behandeln bzw. darauf einzuwirken. Damit könnte beispielsweise die Gerinnung von Blut verhindert oder angestoßen bzw. initiiert werden. Zur Abtrennung von Plasma als leichtere Phase 3 kann z.B. eine Antigerinnungsmittel zumindest bereichsweise auf die Innenfläche 11 aufgebracht werden.

Des Weiteren wäre es aber auch noch möglich, die Beschichtung derart auszubilden, dass das Gleitverhalten des Trennelements 14 für dessen Einsetzvorgang hin zur Bereitschaftsposition und/oder für den nachfolgenden Trenn- bzw. Separiervorgang verbessert ist. Weiters kann die Beschichtung aber auch zwischen dem Trennelement 14 und der Innenfläche 11 der Seitenwand 10 beispielsweise die Gleitreibung verringern bzw. begünstigen und die Haftreibung erhöhen.

Unabhängig davon könnte aber auch das Trennelement 14 mit einer Beschichtung versehen sein, um so das Anhaften von Bestandteilen des Gemisches 2 zu erschweren bzw. überhaupt zu verhindern. So könnte beispielsweise eine Nanobeschichtung oder dergleichen Anwendung finden.

Ist eine zuvor beschriebene Aufnahmevorrichtung 1 fertig bereitgestellt bzw. ausgebildet, kann in den Aufnahmeraum 13 das Gemisch 2, beispielsweise durch einen Blutabnahmevorgang, eingefüllt werden. Anschließend daran wird das eingefüllte Gemisch 2 mit einer darauf einwirkenden Zentrifugalkraft beaufschlagt. Durch die dabei aufgebauten Fliehkräfte wird das Gemisch 2 in die leichtere Phase 3 mit der geringeren Dichte sowie die schwerere Phase 4 mit der dazu höheren Dichte separiert. Während dieses Separiervorgangs werden die Bestandteile der schwereren Phase 4 näher zum Boden 9 hin bewegt, wobei die Bestandteile der leichteren Phase 3 auf jenen der schwereren Phase 4 aufschwimmen. Während des Einwirkens der Zentrifugalkraft wird weiters das Trennelement 14 elastisch soweit verformt, dass das Trennelement 14 bereichsweise von seiner an der Innenfläche 11 der Seitenwand 10 anliegenden, vorgespannt positioniert gehaltenen Bereitschaftsposition von der Seitenwand distanziert wird. So wird zumindest ein Strömungskanal zwischen dem Trennelement 14 und der Innenfläche 11 der Seitenwand 10 des Aufnahmebehälters 5 ausgebildet. Durch die Bildung dieses Strömungskanals können nun die Bestandteile der schwereren Phase 4 hindurchtreten und so in den Bereich des Bodens 9 gelangen. Dadurch, dass die Dichte des Trennelements 14 bezüglich der Bestandteile der schweren Phase 4 dazu geringer gewählt ist, kommt es zu einem Aufschwimmen des Trennelements 14 auf den Bestandteilen der schwereren Phase 4. Durch dieses Aufschwimmen wird das noch immer elastisch verformte Trennelement 14 im Zuge des Trennvorgangs des Gemisches 2 in die zwischen den beiden voneinander separierten Phasen ausgebildete Trennebene selbsttätig verlagert.

Ist eine ausreichende Trennung der beiden Phasen 3, 4 voneinander erfolgt, kann die darauf einwirkende Zentrifugalkraft reduziert und in weiterer Folge vollständig weggenommen werden. Bei einem zumindest teilweise wegnehmen bzw. reduzieren der Zentrifugalkraft wird der zwischen dem Trennelement 14 und der Innenfläche 11 der Seitenwand 10 ausgebildete Strömungskanal durch die elastische Rückstellung des Trennelements 14 selbsttätig dicht verschlossen.

Durch die zuvor beschriebenen Abmessungsunterschiede zwischen dem Trennelement 14 und dem zweiten Teilabschnitt 30 des Aufnahmebehälters 5 kommt es in der Trennstellung ebenfalls zu einer rundum dichtenden Anlage des Trennelements 14 an der Innenfläche 11 der Seitenwand 10.

Um eine ausreichende Trennung des Gemisches 2 in seine beiden Phasen 3, 4 zu erzielen, ist eine entsprechende Zeitspanne der Einwirkung der Zentrifugalkraft zu wählen. Dabei kann die Zeitspanne einige Minuten betragen, wobei bevorzugt eine Zeitspanne von zumindest 10 Minuten gewählt werden kann.

Die Ausführungsbeispiele zeigen mögliche Ausführungsvarianten der Aufnahmevorrichtung 1, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mitumfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1, oder 5,5 bis 10.

Vor allem können die einzelnen in den Fig. 1, 2; 3; 4; 5 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

Der Ordnung halber sei abschließend darauf hingewiesen, dass zum besseren Verständnis des Aufbaus der Aufnahmevorrichtung 1 diese bzw. deren Bestandteile teilweise unmaßstäblich und/oder vergrößert und/oder verkleinert dargestellt wurden. Es wurden auch nur die Verfahrensschritte stark vereinfacht wiedergegeben.

**Bezugszeichenaufstellung**

| | | | |
|---|---|---|---|
| 1 | Aufnahmevorrichtung | 31 | Übergangsabschnitt |
| 2 | Gemisch | 32 | Kanal |
| 3 | leichtere Phase | 33 | Montagerohr |
| 4 | schwerere Phase | 34 | Stirnende |
| 5 | Aufnahmebehälter | 35 | Montagedorn |
| 6 | Längsachse | 36 | Stirnende |
| 7 | erstes Ende | 37 | Unterdruckeinheit |
| 8 | zweites Ende | 38 | Positionierelement |
| 9 | Boden | 39 | Kammer |
| 10 | Seitenwand | | |
| 11 | Innenfläche | | |
| 12 | Außenfläche | | |
| 13 | Aufnahmeraum | | |
| 14 | Trennelement | | |
| 15 | Verschlusseinheit | | |
| 16 | Kappe | | |
| 17 | Dichtelement | | |
| 18 | Dichtfläche | | |
| 19 | Fortsatz | | |
| 20 | Fortsatz | | |
| 21 | Ansatz | | |
| 22 | Haltering | | |
| 23 | Anlageebene | | |
| 24 | Außendurchmesser | | |
| 25 | Querschnittsabmessung | | |
| 26 | Anlagebereich | | |
| 27 | Übergangsbereich | | |
| 28 | Bodenfläche | | |
| 29 | erster Teilabschnitt | | |
| 30 | zweiter Teilabschnitt | | |

## Patentansprüche

1. Aufnahmevorrichtung (1) zum Trennen eines Gemisches (2), insbesondere von Blut, in eine leichtere Phase (3) mit geringerer Dichte und eine schwerere Phase (4) mit einer dazu größeren Dichte, mit
- einem Aufnahmebehälter (5), der in Richtung einer Längsachse (6) voneinander distanziert ein erstes Ende (7) und ein zweites Ende (8) aufweist, wobei das erste Ende (7) offen und das zweite Ende (8) mit einem Boden (9) verschlossen ist, und sich zwischen dem ersten Ende (7) und dem zweiten Ende (8) eine Seitenwand (10) mit einer Innenfläche (11) und einer Außenfläche (12) erstreckt und die Innenfläche (11) der Seitenwand (10) in einem Übergangsbereich (27) in eine durch den Boden (9) gebildete Bodenfläche (28) übergeht, wobei die Seitenwand (10) und der Boden (9) einen Aufnahmeraum (13) umgrenzen,
- einem Trennelement (14), das im Aufnahmeraums (13) angeordnet ist und vollständig sowie durchgängig aus einem elastisch verformbaren Werkstoff gebildet ist, wobei die Dichte des Trennelements (14) zwischen jenen der zu trennenden Phasen (3, 4) liegt,
- einer abnehmbaren Verschlusseinheit (15), die das erste, offene Ende (7) des Aufnahmebehälters (5) verschließt,
- wobei das Trennelement (14) in seiner unverformten Ausgangsform eine überwiegend kugelig ausgebildete Raumform aufweist und vor dem Befüllen des Aufnahmeraums (13) mit dem zu trennenden Gemisch (2) im Bereich des mit dem Boden (9) verschlossenen zweiten Endes (8) angeordnet ist, und zumindest in einer senkrecht bezüglich der Längsachse (6) ausgerichteten Anlageebene (23) an der Innenfläche (11) der Seitenwand (10) anliegt,
- wobei ein Außendurchmesser (24) des Trennelements (14) in seiner unverformten Ausgangsform größer gewählt ist als eine Querschnittsabmessung (25) des Aufnahmeraums (13) in der Anlageebene (23), und dabei das Trennelement (14) an der Innenfläche (11) der Seitenwand (10) in einer vorgespannten Bereitschaftsposition positioniert gehalten ist,
- und wobei ein dem ersten, offenen Ende (7) unmittelbar benachbarter erster Teilabschnitt (29) der Innenfläche (11) nahezu zylindrisch bezüglich der Längsachse (6) ausgebildet ist,
**dadurch gekennzeichnet,**
- **dass** ein zweiter Teilabschnitt (30) der Innenfläche (11) nahezu zylindrisch bezüglich der Längsachse (6) ausgebildet ist und sich dieser zweite Teilabschnitt (30) ausgehend vom Übergangsbereich (27) der Innenfläche (11) zum Boden (9) hin in Richtung auf das erste offene Ende (7) über eine Axiallänge erstreckt, welche zumindest 50%, bevorzugt 60%, des Füllvolumens des Aufnahmeraums (13) entspricht,
- **dass** zwischen dem Boden (9) und dem zweiten Teilabschnitt (30) der Innenfläche (11) zumindest ein Kanal (32) angeordnet oder ausgebildet ist, welcher bei im Aufnahmeraum (13) angeordnetem Trennelement (14) jene beidseits der Anlageebene (23) befindlichen Teilräume des Aufnahmeraums (13) verbindet und
- **dass** der zumindest eine Kanal (32) entweder durch Vertiefungen in der Innenfläche (11) der Seitenwand (10) und/oder durch Stege oder Vorsprünge, die in radialer Richtung über die Innenfläche (11) in Richtung auf die Längsachse (6) vorragend ausgebildet sind, ausgebildet ist.

2. Aufnahmevorrichtung (1) nach Anspruch 1, **dadurch gekennzeichnet, dass** der Außendurchmesser (24) des Trennelements (14) in seiner unverformten Ausgangsform in einem Wertebereich zwischen 1% und 10%, insbesondere zwischen 2% und 5%, größer ausgebildet ist als die Querschnittsabmessung (25) des Aufnahmeraums (13) in der Anlageebene (23).

3. Aufnahmevorrichtung (1) nach Anspruch 1 oder 2, **dadurch gekennzeichnet, dass** das Trennelement (14) eine Shore A Härte aufweist, welche ausgewählt ist aus einem Wertebereich zwischen 7 Shore A und 20 Shore A, insbesondere zwischen 9 Shore A und 12 Shore A, besonders bevorzugt von 10 Shore A.

4. Aufnahmevorrichtung (1) nach einem der vorhergehenden Ansprüche, **dadurch gekennzeichnet, dass** das Trennelement (14) eine Dichte ausgewählt aus einem Wertebereich zwischen 1,02 kg/m³ und 1,09 kg/m³, insbesondere zwischen 1,03 kg/m³ und 1,04 kg/m³, aufweist.

5. Aufnahmevorrichtung (1) nach einem der vorhergehenden Ansprüche, **dadurch gekennzeichnet, dass** der verschlossene Aufnahmeraum (13) des Aufnahmebehälters (5) auf einen bezüglich des Umgebungsdruckes dazu geringeren Druck abgesenkt ist.

6. Aufnahmevorrichtung (1) nach Anspruch 1, **dadurch gekennzeichnet, dass** eine Axiallänge des ersten Teilabschnitts (29) zumindest einer Längserstreckung einer Dichtfläche (18) eines in den Aufnahmeraums (13) eingesetzten Dichtelements (17) der Verschlusseinheit (15) entspricht.

7. Aufnahmevorrichtung (1) nach Anspruch 1, **dadurch gekennzeichnet, dass** sich der zweite Teilabschnitt (30) ausgehend vom Übergangsbereich (27) der Innenfläche (11) zum Boden (9) bis zum ersten Teilabschnitt (29) erstreckt.

8. Aufnahmevorrichtung (1) nach einem der Ansprüche 1, 6 oder 7, **dadurch gekennzeichnet, dass** der erste Teilabschnitt (29) einen Durchmesser aufweist, welcher bezüglich des zweiten Teilabschnitts (30) dazu größer ausgebildet ist.

9. Aufnahmevorrichtung (1) nach einem der Ansprüche 1 oder 6 bis 8, **dadurch gekennzeichnet, dass** anschließend an den ersten Teilabschnitt (29) auf der dem Boden (9) zugewendeten Seite ein kegelförmig verjüngend ausgebildeten Übergangsabschnitt (31) ausgebildet ist.

10. Verfahren zum Bereitstellen einer Aufnahmevorrichtung zum Trennen eines Gemisches (2), insbesondere von Blut, in eine leichtere Phase (3) mit geringerer Dichte und eine schwerere Phase (4) mit einer dazu größeren Dichte, bei dem
- ein Aufnahmebehälter (5), mit in Richtung einer Längsachse (6) voneinander distanzierten ersten und zweiten Enden (7, 8) ausgebildet wird, wobei das erste Ende (7) offen und das zweite Ende (8) mit einem Boden (9) verschlossen wird, und zwischen dem ersten und dem zweiten Ende (7, 8) eine Seitenwand (10) mit einer Innenfläche (11) und einer Außenfläche (12) ausgebildet wird, wobei von der Seitenwand (10) und dem Boden (9) ein Aufnahmeraum (13) umgrenzt wird,
- ein Trennelement (14) vollständig/durchgängig aus einem elastisch verformbaren Werkstoff gebildet und im Aufnahmeraum (13) angeordnet wird, wobei die Dichte des Trennelements (14) zwischen jenen der zu trennenden Phasen (3, 4) gewählt wird, und
- das erste offene Ende des Aufnahmebehälters (5) mit einer abnehmbaren Verschlusseinheit (15) verschlossen wird,
- wobei das Trennelement (14) mit einer überwiegend kugeligen Raumform ausgebildet wird und das Trennelement (14) noch vor dem Befüllen des Aufnahmeraums (13) mit dem zu trennenden Gemisch (2) zu dem im Bereich des mit dem Boden (9) verschlossenen zweiten Endes (8) verbracht wird und dabei in einer senkrecht bezüglich der Längsachse (6) ausgerichteten Anlageebene (23) an der Innenfläche (11) der Seitenwand (10) zur Anlage gebracht wird,
- wobei bei der Herstellung des Trennelements (14) ein Außendurchmesser (24) des Trennelements (14) in seiner unverformten Ausgangsform größer ausgebildet wird als eine Querschnittsabmessung (25) des Aufnahmeraums (13) in der Anlageebene (23) und so das Trennelement (14) an der Innenfläche (11) der Seitenwand (10) in einer vorgespannten Bereitschaftsposition positioniert gehalten wird,
**dadurch gekennzeichnet,**
- **dass** das Trennelement (14) vor dem Einsetzen in den Aufnahmeraum (13) in ein Montagerohr (33) mit einem bezüglich einer lichten Innenabmessung des Aufnahmeraums (13) dazu geringeren Außenabmessung eingebracht wird, anschließend das Montagerohr (33) soweit in den Aufnahmeraum (13) verbracht wird, dass dessen Stirnende (34) dem Boden (9) benachbart angeordnet ist und anschließend das Trennelement (14) aus dem Montagerohr (33) herausgedrückt wird.

11. Verfahren zum Bereitstellen einer Aufnahmevorrichtung zum Trennen eines Gemisches (2), insbesondere von Blut, in eine leichtere Phase (3) mit geringerer Dichte und eine schwerere Phase (4) mit einer dazu größeren Dichte, bei dem
- ein Aufnahmebehälter (5), mit in Richtung einer Längsachse (6) voneinander distanzierten ersten und zweiten Enden (7, 8) ausgebildet wird, wobei das erste Ende (7) offen und das zweite Ende (8) mit einem Boden (9) verschlossen wird, und zwischen dem ersten und dem zweiten Ende (7, 8) eine Seitenwand (10) mit einer Innenfläche (11) und einer Außenfläche (12) ausgebildet wird, wobei von der Seitenwand (10) und dem Boden (9) ein Aufnahmeraum (13) umgrenzt wird,
- ein Trennelement (14) vollständig/durchgängig aus einem elastisch verformbaren Werkstoff gebildet und im Aufnahmeraum (13) angeordnet wird, wobei die Dichte des Trennelements (14) zwischen jenen der zu trennenden Phasen (3, 4) gewählt wird, und
- das erste offene Ende des Aufnahmebehälters (5) mit einer abnehmbaren Verschlusseinheit (15) verschlossen wird,
- wobei das Trennelement (14) mit einer überwiegend kugeligen Raumform ausgebildet wird und das Trennelement (14) noch vor dem Befüllen des Aufnahmeraums (13) mit dem zu trennenden Gemisch (2) zu dem im Bereich des mit dem Boden (9) verschlossenen zweiten Endes (8) verbracht wird und dabei in einer senkrecht bezüglich der Längsachse (6) ausgerichteten Anlageebene (23) an der Innenfläche (11) der Seitenwand (10) zur Anlage gebracht wird,
- wobei bei der Herstellung des Trennelements (14) ein Außendurchmesser (24) des Trennelements (14) in seiner unverformten Ausgangsform größer ausgebildet wird als eine Querschnittsabmessung (25) des Aufnahmeraums (13) in der Anlageebene (23) und so das Trennelement (14) an der Innenfläche (11) der Seitenwand (10) in einer vorgespannten Bereitschaftsposition positioniert gehalten wird,
**dadurch gekennzeichnet,**
- **dass** das Trennelement (14) von einem hohl ausgebildeten Montagedorn (35) durchstochen wird, dann das Trennelement (14) an der Innenfläche (11) der Seitenwand (10) dichtenden angelegt wird und der Montagedorn (35) soweit in den Aufnahmeraums (13) hinein geschoben wird, dass dessen Stirnende (36) dem Boden (9) benachbart angeordnet ist und dann durch den hohlen Montagedorn (35) der zwischen dem Boden (9) und dem Trennelement (14) abgedichtete Aufnahmeraum (13) auf einen bezüglich des äußeren Umgebungsdruckes dazu geringeren Druck abgesenkt wird, wobei durch den dabei aufgebauten Druckunterschied zwischen dem Aufnahmeraums (13) und der äußeren Umgebung das Trennelement (14) am Montagedorn (35) gleitend zu dem zweiten, durch den Boden (9) verschlossenen Ende (8) verlagert wird.

12. Verfahren zum Bereitstellen einer Aufnahmevorrichtung zum Trennen eines Gemisches (2), insbesondere von Blut, in eine leichtere Phase (3) mit geringerer Dichte und eine schwerere Phase (4) mit einer dazu größeren Dichte, bei dem
- ein Aufnahmebehälter (5), mit in Richtung einer Längsachse (6) voneinander distanzierten ersten und zweiten Enden (7, 8) ausgebildet wird, wobei das erste Ende (7) offen und das zweite Ende (8) mit einem Boden (9) verschlossen wird, und zwischen dem ersten und dem zweiten Ende (7, 8) eine Seitenwand (10) mit einer Innenfläche (11) und einer Außenfläche (12) ausgebildet wird, wobei von der Seitenwand (10) und dem Boden (9) ein Aufnahmeraum (13) umgrenzt wird,
- ein Trennelement (14) vollständig/durchgängig aus einem elastisch verformbaren Werkstoff gebildet und im Aufnahmeraums (13) angeordnet wird, wobei die Dichte des Trennelements (14) zwischen jenen der zu trennenden Phasen (3, 4) gewählt wird, und
- das erste offene Ende des Aufnahmebehälters (5) mit einer abnehmbaren Verschlusseinheit (15) verschlossen wird,
- wobei das Trennelement (14) mit einer überwiegend kugeligen Raumform ausgebildet wird und das Trennelement (14) noch vor dem Befüllen des Aufnahmeraums (13) mit dem zu trennenden Gemisch (2) zu dem im Bereich des mit dem Boden (9) verschlossenen zweiten Endes (8) verbracht wird und dabei in einer senkrecht bezüglich der Längsachse (6) ausgerichteten Anlageebene (23) an der Innenfläche (11) der Seitenwand (10) zur Anlage gebracht wird,
- wobei bei der Herstellung des Trennelements (14) ein Außendurchmesser (24) des Trennelements (14) in seiner unverformten Ausgangsform größer ausgebildet wird als eine Querschnittsabmessung (25) des Aufnahmeraums (13) in der Anlageebene (23) und so das Trennelement (14) an der Innenfläche (11) der Seitenwand (10) in einer vorgespannten Bereitschaftsposition positioniert gehalten wird,
**dadurch gekennzeichnet,**
- **dass** vor dem Verschließen des Aufnahmebehälters (5) mit der Verschlusseinheit (15) der den Aufnahmebehälter (5) umgebende Bereich auf einen bezüglich des äußeren Umgebungsdruckes dazu geringeren Druck abgesenkt wird, dann das Trennelement (14) in das erste offene Ende (7) des Aufnahmebehälters (5) verbracht und dort zur Anlage an der Innenfläche (11) gebracht wird, nachfolgend der Aufnahmebehälter (5) einem dazu höheren Druck, insbesondere dem äußeren Umgebungsdruck, ausgesetzt wird und durch den dabei aufgebauten Druckunterschied zwischen dem Aufnahmeraum (13) und dem das Trennelement (14) umgebenden Raum zu dem zweiten, durch den Boden (9) verschlossenen Ende (8) verlagert wird.

13. Verfahren nach einem der Ansprüche 10 bis 12, **dadurch gekennzeichnet, dass** der Aufnahmeraum (13) des Aufnahmebehälters (5) auf einen bezüglich des Umgebungsdruckes dazu geringeren Druck abgesenkt und anschließend die Verschlusseinheit (15) auf den Aufnahmebehälter (5) aufgesetzt wird.

14. Verfahren zum Trennen eines Gemisches (2), insbesondere von Blut, in eine leichtere Phase (3) mit geringerer Dichte und eine schwerere Phase (4) mit einer dazu größeren Dichte, unter Verwendung einer Aufnahmevorrichtung nach den Ansprüchen 1 bis 9 und/oder einer nach dem Verfahren bereitgestellten Aufnahmevorrichtung gemäß den Ansprüchen 10 bis 13, bei dem in den von der Verschlusseinheit (15) gegenüber der äußeren Umgebung abgeschlossenen Aufnahmeraum (13) das zu trennende Gemisch (2), insbesondere Blut, eingefüllt wird, anschließend das eingefüllte Gemisch (2) mit einer darauf einwirkenden Zentrifugalkraft beaufschlagt wird, und dabei das Gemisch (2) in die Phase (3) mit der geringeren Dichte und jene Phase (4) mit der dazu höheren Dichte separiert wird, wobei während des Einwirkens der Zentrifugalkraft das Trennelement (14) elastisch soweit verformt wird, dass das Trennelement (14) bereichsweise von seiner an der Innenfläche (11) der Seitenwand (10) anliegenden, vorgespannt positioniert gehaltenen Bereitschaftsposition von der Seitenwand (10) distanziert wird, und dabei ein Strömungskanal zwischen dem Trennelement (14) und der Innenfläche (11) der Seitenwand (10) des Aufnahmebehälters (5) ausgebildet wird, und weiters das elastisch verformte Trennelement (14) im Zuge des Trennvorgangs des Gemisches (2) in die zwischen den beiden voneinander separierten Phasen (3, 4) ausgebildete Trennebene selbsttätig verlagert wird, wobei nach der zumindest teilweisen Wegnahme der Zentrifugalkraft der zwischen dem Trennelement (14) und der Innenfläche (11) der Seitenwand (10) ausgebildete Strömungskanal durch die elastische Rückstellung des Trennelements (14) dicht verschlossen wird.

15. Verfahren nach Anspruch 14, **dadurch gekennzeichnet, dass** das Gemisch (2) zumindest über eine Zeitspanne von 10 min der Zentrifugalkraft ausgesetzt wird.

## Claims

1. A receptacle device (1) for separating a mixture (2), in particular blood, into a lighter phase (3) with a lower density and a heavier phase (4) with a relatively higher density, comprising
- a receptacle (5) having a first end (7) and a second end (8) spaced apart from one another in the direction of a longitudinal axis (6), the first end (7) being open and the second end (8) being closed by a base (9), and a side wall (10) having an internal face (11) and an external face (12) extends between the first end (7) and the second end (8), and the internal face (11) of the side wall (10) develops into a base surface (28) formed by the base (9) in a transition region (27), wherein the side wall (10) and the base (9) delineate a receptacle chamber (13),
- a separating element (14), which is disposed in the receptacle chamber (13) and is made entirely and continuously of an elastically deformable material, the density of the separating element (14) being between the densities of the phases (3, 4) to be separated,
- a removable closure unit (15), which closes the first, open end (7) of the receptacle (5),
- wherein the separating element (14) in its undeformed initial state, has a predominantly spherical three-dimensional shape and is disposed in the region of the second end (8), which is closed by the base (9), before the receptacle chamber (13) is filled with the mixture to be separated (2) and is in contact with the internal face (11) of the side wall (10) at least in a contact plane (23) oriented perpendicular to the longitudinal axis (6),
- an external diameter (24) of the separating element (14) being selected to be larger in its undeformed initial state than a cross-sectional dimension (25) of the receptacle chamber (13) in the contact plane (23), and the separating element (14) is thus held in position against the internal face (11) of the side wall (10) in a prestressed standby position,
- and a first part section (29) of the internal face (11), which is directly adjacent to the first open end (7) is designed to be almost cylindrical with respect to the longitudinal axis (6),
**characterized in that**
- a second part section (30) of the internal face (11) is designed to be almost cylindrical with respect to the longitudinal axis (6), and this second part section (30) extends over an axial length in the direction towards the first open end (7), starting from the transition region (27) of the internal face (11) to the base (9), said axial length corresponding to at least 50%, preferably 60% of the filling volume of the receptacle chamber (13),
- at least one passage (32) is disposed or formed between the base (9) and the second part section (30) of the internal face (11), said passage (32) connecting partial chambers of the receptacle chamber (13) disposed on both sides of the contact plane (23) when the separating element (14) is disposed in the receptacle chamber (13), and
- the at least one passage (32) is formed either by recesses in the internal face (11) of the side wall (10) and/or by webs or protrusions formed to protrude in the radial direction over the internal face (11) in the direction towards the longitudinal axis (6).

2. The receptacle device (1) according to claim 1, **characterized in that** the external diameter (24) of the separating element (14) in its undeformed initial state is designed to be larger than the cross-sectional dimension (25) of the receptacle chamber (13) in the contact plane (23) in a range of values between 1% and 10%, in particular between 2% and 5%.

3. The receptacle device (1) according to claim 1 or 2, **characterized in that** the separating element (14) has a Shore A hardness, selected from a range of values between Shore A 7 and Shore A 20, in particular between Shore A 9 and Shore A 12, particularly preferably Shore A 10.

4. The receptacle device (1) according to one of the preceding claims, **characterized in that** the separating element (14) has a density, selected from a range of values between 1.02 kg/m³ and 1.09 kg/m³, in particular between 1.03 kg/m³ and 1.04 kg/m³.

5. The receptacle device (1) according to one of the preceding claims, **characterized in that** the pressure in the closed receptacle chamber (13) of the receptacle (5) is reduced to a pressure below atmospheric pressure.

6. The receptacle device (1) according to claim 1, **characterized in that** an axial length of the first part section (29) corresponds to at least one longitudinal extent of a sealing surface (18) of a seal element (17) of the closure unit (15) inserted into the receptacle chamber (13).

7. The receptacle device (1) according to claim 1, **characterized in that** the second part section (30) of the internal face (11) extends from the transition region (27) from the internal face (11) to the base (9) as far as the first part section (29).

8. The receptacle device (1) according to one of claims 1, 6 or 7, **characterized in that** the first part section (29) has a diameter which is designed to be larger than that of the second part section (30).

9. The receptacle device (1) according to one of claims 1 or 6 to 8, **characterized in that** a conically tapering transition section (31) is designed to connect to the first part section (29) on the side facing the base (9).

10. A method for providing a receptacle device for separating a mixture (2), in particular blood, into a lighter phase (3) with a lower density and a heavier phase (4) with a relatively higher density, in which
- a receptacle (5) having a first end (7) and second end (8) spaced a distance apart from one another in the direction of a longitudinal axis (6) is provided, the first end (7) being open and the second end (8) being closed by a base (9), and a side wall (10) with an internal face (11) and an external face (12) being designed between the first end (7) and the second end (8), a receptacle chamber (13) being delineated by the side wall (10) and the base (9),
- a separating element (14) is made entirely and continuously of an elastically deformable material and is disposed in the receptacle chamber (13), the density of the separating element (14) being selected so that it is between that of the phases (3, 4) to be separated, and
- the first open end of the receptacle (5) is closed by a removable closure unit (15),
- wherein the separating element (14) is designed with a predominantly spherical three-dimensional shape, and the separating element (14) is moved toward the region of the second end (8) which is closed by the base (9) before the receptacle chamber (13) is filled with the mixture (2) to be separated and is therefore in contact with the internal face (11) of the side wall (10) in a contact plane (23) oriented perpendicular to the longitudinal axis (6),
- wherein, when manufacturing the separating element (14), an external diameter (24) of the separating element (14) is designed to be larger in its undeformed initial state than a cross-sectional dimension (25) of the receptacle chamber (13) in the contact plane (23) and therefore the separating element (14) is held in a prestressed standby position against the internal face (11) of the side wall (10),
**characterized in that**
- before being inserted in the receptacle chamber (13), the separating element (14) is introduced into a fitting tube (33) having an internal dimension of the receptacle chamber (13) that is smaller than a clear internal dimension, after which the fitting tube (33) is moved into the receptacle chamber (13) to the extent that its end face (34) is disposed adjacent to the base (9), and the separating element (14) is then forced out of the fitting tube (33).

11. The method for providing a receptacle device for separating a mixture (2), in particular blood, into a lighter phase (3) with a lower density and a heavier phase (4) with a higher density, in which
- a receptacle (5) having a first end (7) and second end (8) spaced a distance apart from one another in the direction of a longitudinal axis (6) is provided, the first end (7) being open and the second end (8) being closed by a base (9), and a side wall (10) with an internal face (11) and an external face (12) being designed between the first end (7) and the second end (8), a receptacle chamber (13) being delineated by the side wall (10) and the base (9),
- a separating element (14) is made entirely and continuously of an elastically deformable material and is disposed in the receptacle chamber (13), the density of the separating element (14) being selected to be between that of the phases (3, 4) to be separated, and
- the first open end of the receptacle (5) is closed by a removable closure unit (15),
- wherein the separating element (14) is designed with a predominantly spherical three-dimensional shape, and the separating element (14) is moved toward the region of the second end (8), which is closed by the base (9), before the receptacle chamber (13) is filled with the mixture (2) to be separated and is therefore in contact with the internal face (11) of the side wall (10) in a contact plane (23) oriented perpendicular to the longitudinal axis (6),
- wherein, in the manufacture of the separating element (14), an external diameter (24) of the separating element (14) is designed to be larger in its undeformed initial state than a cross-sectional dimension (25) of the receptacle chamber (13) in the contact plane (23), and therefore the separating element (14) is held in a prestressed standby position against the internal face (11) of the side wall (10),
**characterized in that**
- the separating element (14) is pierced by a hollow assembly pin (35), then the separating element (14) is placed against the internal face (11) of the side wall (10) in a sealing arrangement, and the assembly pin (35) is pushed into the receptacle chamber (13) to the extent that its end face (36) is disposed adjacent to the base (9), and then the pressure in the sealed receptacle chamber (13) between the base (9) and the separating element (14) is reduced through the hollow assembly pin (35) to a pressure lower than the outside ambient pressure, and due to the pressure difference created between the receptacle chamber (13) and the outside environment, the separating element (14) is moved on the assembly pin (35) in a sliding movement to the second end (8), which is closed by the base (9).

12. The method for providing a receptacle device for separating a mixture (2), in particular blood, into a lighter phase (3) with a lower density and a heavier phase (4) with a higher density, in which
- a receptacle (5) having a first end (7) and second end (8) spaced a distance apart from one another in the direction of a longitudinal axis (6) is provided, the first end (7) being open and the second end (8) being closed by a base (9), and a side wall (10) with an internal face (11) and an external face (12) being designed between the first end (7) and the second end (8), a receptacle chamber (13) being delineated by the side wall (10) and the base (9),
- a separating element (14) is made entirely and continuously of an elastically deformable material and is disposed in the receptacle chamber (13), the density of the separating element (14) being selected to be between that of the phases (3, 4) to be separated, and
- the first open end of the receptacle (5) is closed by a removable closure unit (15),
- wherein the separating element (14) is designed with a predominantly spherical three-dimensional shape, and the separating element (14) is moved toward the region of the second end (8), which is closed by the base (9), before the receptacle chamber (13) is filled with the mixture (2) to be separated and is therefore in contact with the internal face (11) of the side wall (10) in a contact plane (23) oriented perpendicular to the longitudinal axis (6),
- wherein, in the manufacture of the separating element (14), an external diameter (24) of the separating element (14) is designed to be larger in its undeformed initial state than a cross-sectional dimension (25) of the receptacle chamber (13) in the contact plane (23), and therefore the separating element (14) is held in a prestressed standby position against the internal face (11) of the side wall (10),
**characterized in that**
- before closing the receptacle (5) with the closure unit (15), the pressure in the area surrounding the receptacle (5) is lowered to a pressure below the outside ambient pressure, then the separating element (14) is placed in the first open end (7) of the receptacle (5) and moved so that it is in contact with the internal face (11), after which the receptacle (5) is exposed to a higher pressure, in particular to the outside ambient pressure, and due to the resulting pressure difference created between the receptacle chamber (13) and the area surrounding the separating element (14), the latter is moved to the second end (8) which is closed by the base (9).

13. The method according to one of claims 10 to 12, **characterized in that** the pressure in the receptacle chamber (13) of the receptacle (5) is reduced to a pressure below atmospheric pressure, and then the closure unit (15) is placed on the receptacle (5).

14. The method for separating a mixture (2), in particular blood, into a lighter phase (3) with a lower density and a heavier phase (4) with a higher density, using a receptacle device according to claims 1 to 9 and/or a receptacle device provided by the method according to claims 10 to 13, in which the receptacle chamber (13), which is closed off from the outside environment by the closure unit (15), is filled with the mixture (2), in particular blood, to be separated, and the mixture (2) in the chamber is then subjected to a centrifugal force that acts on it, thereby separating the mixture (2) into the phase (3) with the lower density and the phase (4) with the higher density, such that under the influence of the centrifugal force, the separating element (14) is elastically deformed to the extent that certain regions of the separating element (14) are moved from the standby position, in which it is held in prestressed form in contact with the internal face (11) of the side wall (10), to a position at a distance from the side wall (10), and a flow passage is thereby created between the separating element (14) and the internal face (11) of the side wall (10) of the receptacle (5), and furthermore, the elastically deformed separating element (14) is automatically displaced into a separation plane formed between the two mutually separated phases (3, 4) during the process of separating the mixture (2), and after at least partially removing the centrifugal force, the flow passage thereby formed between the separating element (14) and the internal face (11) of the side wall (10) is closed tightly by the separating element (14) due to the elastic rebound.

15. The method according to claim 14, **characterized in that** the mixture (2) is subjected to the centrifugal force for a period of at least 10 minutes.

## Revendications

1. Dispositif de logement (1) pour la séparation d'un mélange (2), plus particulièrement de sang, en une phase légère (3) avec une faible densité et une phase lourde (4) avec une densité plus importante, avec
- un récipient (5), qui comprend, éloignées entre elles en direction d'un axe longitudinal (6), une première extrémité (7) et une deuxième extrémité (8), la première extrémité (7) étant ouverte et la deuxième extrémité (8) étant obturée par un fond (9), et une paroi latérale (10) avec une surface interne (11) et une surface externe (12) s'étendant entre la première extrémité (7) et la deuxième extrémité (8), et la surface interne (11) de la paroi latérale (10) se transformant, dans une zone de transition (27), en une surface de fond (28) constituée par le fond (9), la paroi latérale (10) et le fond (9) délimitant un espace de logement (13),
- un élément de séparation (14), qui est disposé dans l'espace de logement (13) et qui est constitué entièrement ainsi que de manière continue d'un matériau déformable élastiquement, la densité de l'élément de séparation (14) se trouvant entre celles des phases (3, 4) à séparer,
- une unité d'obturation amovible (15), qui obture la première extrémité ouverte (7) du récipient (5),
- l'élément de séparation (14) comprenant, dans sa forme initiale non déformée, une forme spatiale globalement sphérique et étant disposé, avant le remplissage de l'espace de logement (13) avec le mélange à séparer (2), au niveau de la deuxième extrémité (8) obturée avec le fond (9), et s'appuyant, au moins dans un plan d'appui (23) orienté perpendiculairement par rapport à l'axe longitudinal (6), contre la surface interne (11) de la paroi latérale (10),
- un diamètre extérieur (24) de l'élément de séparation (14) dans sa forme initiale non déformée étant choisi supérieur à une dimension de section transversale (25) de l'espace de logement (13) dans le plan d'appui (23) et l'élément de séparation (14) étant maintenu contre la surface interne (11) de la paroi latérale (10) dans une position d'attente précontrainte,
- et une première portion (29) de la surface interne (11), directement adjacente à la première extrémité ouverte (7), étant conçue de manière presque cylindrique par rapport à l'axe longitudinal (6),
**caractérisé en ce que**
- une deuxième portion (30) de la surface interne (11) étant conçue de manière presque cylindrique par rapport à l'axe longitudinal (6), et cette deuxième portion (30) s'étendant de la zone de transition (27) de la surface interne (11) vers le fond (9) en direction de la première extrémité ouverte (7) sur une longueur axiale qui correspond à au moins 50 %, de préférence 60 %, de la capacité de remplissage de l'espace de logement (13),
- entre le fond (9) et la deuxième portion (30) de la surface interne (11) est disposé ou réalisé au moins un canal (32) qui relie, lorsque l'élément de séparation (14) est disposé dans l'espace de logement (13), les deux espaces partiels de l'espace de logement (13) se trouvant des deux côtés du plan d'appui (23) et
- l'au moins un canal (32) est constitué par des cavités dans la surface interne (11) de la paroi latérale (10) et/ou par des nervures ou des saillies qui sont conçues de façon à dépasser de la surface interne (11) en direction de l'axe longitudinal (6).

2. Dispositif de logement (1) selon la revendication 1, **caractérisé en ce que** le diamètre extérieur (24) de l'élément de séparation (14) dans sa forme initiale non déformée est supérieur, dans une plage de valeurs entre 1 % et 10 %, plus particulièrement entre 2 % et 5 %, à la dimension de section transversale (25) de l'espace de logement (13) dans le plan d'appui (23).

3. Dispositif de logement (1) selon la revendication 1 ou 2, **caractérisé en ce que** l'élément de séparation (14) présente une dureté Shore A qui est sélectionnée dans une plage de valeurs entre 7 Shore A et 20 Shore A, plus particulièrement entre 9 Shore A et 12 Shore A, plus particulièrement de préférence de 10 Shore A.

4. Dispositif de logement (1) selon l'une des revendications précédentes, **caractérisé en ce que** l'élément de séparation (14) présente une densité sélectionnée dans une plage de valeurs entre 1,02 kg/m³ et 1,09 kg/m³, plus particulièrement entre 1,03 kg/m³ et 1,04 kg/m³.

5. Dispositif de logement (1) selon l'une des revendications précédentes, **caractérisé en ce que** l'espace de logement obturé (13) du récipient (5) est réduit à une pression inférieure à la pression ambiante.

6. Dispositif de logement (1) selon la revendication 1, **caractérisé en ce qu'**une longueur axiale de la première portion (29) correspond au moins à une extension longitudinale d'une surface d'étanchéité (18) d'un élément d'étanchéité (17), inséré dans l'espace de logement (13), de l'unité d'obturation (15).

7. Dispositif de logement (1) selon la revendication 1, **caractérisé en ce que** la deuxième portion (30) s'étend de la zone de transition (27) de la surface interne (11) vers le fond (9) jusqu'à la première portion (29).

8. Dispositif de logement (1) selon l'une des revendications 1, 6 ou 7, **caractérisé en ce que** la première portion (29) présente un diamètre qui est supérieur à celui de la deuxième portion (30).

9. Dispositif de logement (1) selon l'une des revendications 1 ou 6 à 8, **caractérisé en ce que**, après la première portion (29), sur le côté orienté vers le fond (9), est réalisée une portion de transition (31) se rétrécissant de manière conique.

10. Procédé de réalisation d'un dispositif de logement pour la séparation d'un mélange (2), plus particulièrement de sang, en une phase légère (3) avec une faible densité et une phase lourde (4) avec une densité plus importante, dans lequel
- un récipient (5), avec une première et une deuxième extrémité (7, 8), éloignées entre elles dans la direction d'un axe longitudinal (6), est réalisé, la première extrémité (7) étant ouverte et la deuxième extrémité (8) étant obturée par un fond (9) et une paroi latérale (10) avec une surface interne (11) et une surface externe (12) s'étendant entre la première extrémité (7) et la deuxième extrémité (8), la paroi latérale (10) et le fond (9) délimitant un espace de logement (13),
- un élément de séparation (14) est constitué entièrement / de manière continue d'un matériau déformable élastiquement et est disposé dans l'espace de logement (13), la densité de l'élément de séparation (14) se trouvant entre celles des phases (3, 4) à séparer, et
- la première extrémité ouverte du récipient (5) est obturé par une unité d'obturation amovible (15),
- l'élément de séparation (14) étant réalisé avec une forme spatiale globalement sphérique et l'élément de séparation (14) étant amené, avant le remplissage de l'espace de logement (13) avec le mélange à séparer (2), au niveau de la deuxième extrémité (8) obturée par le fond (9) et étant donc mis en appui, dans un plan d'appui (23) orienté perpendiculairement par rapport à l'axe longitudinal (6), contre la surface interne (11) de la paroi latérale (10),
- lors de la fabrication de l'élément de séparation (14), un diamètre extérieur (24) de l'élément de séparation (14) dans sa forme initiale non déformée étant supérieure à une dimension de section transversale (25) de l'espace de logement (13) dans le plan d'appui (23) et l'élément de séparation (14) étant ainsi maintenu contre la surface interne (11) de la paroi (10) dans une position d'attente précontrainte,
**caractérisé en ce que**
- l'élément de séparation (14) est inséré, avant l'insertion dans l'espace de logement (13), dans un tube de montage (33) avec une dimension inférieure à une dimension interne de l'espace de logement (13), puis le tube de montage (33) est amené dans l'espace de logement (13) jusqu'à ce que son extrémité frontale (34) soit adjacente au fond (9) puis l'élément de séparation (14) est expulsé du tube de montage (33).

11. Procédé de réalisation d'un dispositif de logement pour la séparation d'un mélange (2), plus particulièrement de sang, en une phase légère (3) avec une faible densité et une phase lourde (4) avec une densité plus importante, dans lequel
- un récipient (5), avec une première et une deuxième extrémité (7, 8), éloignées entre elles en direction d'un axe longitudinal (6), est réalisé, la première extrémité (7) étant ouverte et la deuxième extrémité (8) étant obturée par un fond (9) et une paroi latérale (10) avec une surface interne (11) et une surface externe (12) s'étendant entre la première extrémité (7) et la deuxième extrémité (8), la paroi latérale (10) et le fond (9) délimitant un espace de logement (13),
- un élément de séparation (14) est constitué entièrement / de manière continue d'un matériau déformable élastiquement et est disposé dans l'espace de logement (13), la densité de l'élément de séparation (14) se trouvant entre celles des phases (3, 4) à séparer, et
- la première extrémité ouverte du récipient (5) est obturé par une unité d'obturation amovible (15),
- l'élément de séparation (14) étant réalisé avec une forme spatiale globalement sphérique et l'élément de séparation (14) étant amené, avant le remplissage de l'espace de logement (13) avec le mélange à séparer (2), au niveau de la deuxième extrémité (8) obturée par le fond (9) et étant donc mis en appui, dans un plan d'appui (23) orienté perpendiculairement par rapport à l'axe longitudinal (6), contre la surface interne (11) de la paroi latérale (10),
- lors de la fabrication de l'élément de séparation (14), un diamètre extérieur (24) de l'élément de séparation (14) dans sa forme initiale non déformée étant supérieure à une dimension de section transversale (25) de l'espace de logement (13) dans le plan d'appui (23) et l'élément de séparation (14) étant ainsi maintenu contre la surface interne (11) de la paroi latérale (10) dans une position d'attente précontrainte,
**caractérisé en ce que**
- l'élément de séparation (14) est transpercé par un mandrin de montage creux (35), puis l'élément de séparation (14) est appuyé de manière étanche contre la surface interne (11) de la paroi latérale (10) et le mandrin de montage (35) est poussé dans l'espace de logement (13) jusqu'à ce que son extrémité frontale (36) soit adjacente au fond (9) puis, grâce au mandrin de montage (35), l'espace de logement (13) étanchéifié entre le fond (9) et l'élément de séparation (14), est réduit à une pression inférieure à la pression ambiante, la différence de pression ainsi générée entre l'espace de logement (13) et l'environnement extérieur permettant de faire coulisser l'élément de séparation (14) le long du mandrin de montage (35) vers la deuxième extrémité (8) obturée par le fond (9).

12. Procédé de réalisation d'un dispositif de logement pour la séparation d'un mélange (2), plus particulièrement de sang, en une phase légère (3) avec une faible densité et une phase lourde (4) avec une densité plus importante, dans lequel
- un récipient (5), avec une première et une deuxième extrémité (7, 8), éloignées entre elles en direction d'un axe longitudinal (6), est réalisé, la première extrémité (7) étant ouverte et la deuxième extrémité (8) étant obturée par un fond (9) et une paroi latérale (10) avec une surface interne (11) et une surface externe (12) s'étendant entre la première extrémité (7) et la deuxième extrémité (8), la paroi latérale (10) et le fond (9) délimitant un espace de logement (13),
- un élément de séparation (14) est constitué entièrement / de manière continue d'un matériau déformable élastiquement et est disposé dans l'espace de logement (13), la densité de l'élément de séparation (14) se trouvant entre celles des phases (3, 4) à séparer, et
- la première extrémité ouverte du récipient (5) est obturé par une unité d'obturation amovible (15),
- l'élément de séparation (14) étant réalisé avec une forme spatiale globalement sphérique et l'élément de séparation (14) étant amené, avant le remplissage de l'espace de logement (13) avec le mélange à séparer (2), au niveau de la deuxième extrémité (8) obturée par le fond (9) et étant donc mis en appui, dans un plan d'appui (23) orienté perpendiculairement par rapport à l'axe longitudinal (6), contre la surface interne (11) de la paroi latérale (10),
- lors de la fabrication de l'élément de séparation (14), un diamètre extérieur (24) de l'élément de séparation (14) dans sa forme initiale non déformée étant supérieure à une dimension de section transversale (25) de l'espace de logement (13) dans le plan d'appui (23) et l'élément de séparation (14) étant ainsi maintenu contre la surface interne (11) de la paroi latérale (10) dans une position d'attente précontrainte,
**caractérisé en ce que**
- avant l'obturation du récipient (5) avec l'unité d'obturation (15), la zone entourant le récipient (5) est réduite à une pression inférieure à la pression extérieure ambiante, puis l'élément de séparation (14) est amené dans la première extrémité ouverte (7) du récipient (5) puis amené en appui contre la surface interne (11), puis le récipient (5) est exposé à une pression plus élevée, plus particulièrement à la pression extérieure ambiante et la différence de pression ainsi générée entre l'espace de logement (13) et l'espace entourant l'élément de séparation (14) permettant de le déplacer vers la deuxième extrémité (8), obturée par le fond (9).

13. Procédé selon l'une des revendications 10 à 12, **caractérisé en ce que** l'espace de logement (13) du récipient (5) est réduit à une pression inférieure à la pression ambiante puis l'unité d'obturation (15) est posée sur le récipient (5).

14. Procédé de séparation d'un mélange (2), plus particulièrement de sang, en une phase légère (3) de faible densité et une phase lourde (4) d'une densité plus importante, à l'aide d'un dispositif de logement selon les revendications 1 à 9 et/ou un dispositif de logement, réalisé selon le procédé selon les revendications 10 à 13, dans lequel le mélange à séparer (2), plus particulièrement du sang, est introduit dans l'espace de logement (13) obturé par l'unité d'obturation (15) par rapport à l'environnement extérieur, puis le mélange (2) introduit est exposé à une force centrifuge et le mélange (2) est ainsi séparé dans la phase (3) de faible densité et la phase (4) de densité plus importante, l'élément de séparation (14) étant déformé élastiquement pendant l'action de la force centrifuge de façon à ce que l'élément de séparation (14) soit éloigné partiellement de sa position d'attente maintenu de manière précontrainte en appui contre la surface interne (11) de la paroi latérale (10) et un canal d'écoulement est réalisé entre l'élément de séparation (14) et la surface interne (11) de la paroi latérale (10) du récipient (5), et l'élément de séparation (14) déformé élastiquement est en outre déplacé automatiquement, au cours du processus de séparation du mélange (2), vers le plan de séparation formé entre les deux phases séparées (3,4), le canal d'écoulement formé entre l'élément de séparation (14) et la surface interne (11) de la paroi latérale (10) étant obturé de manière étanche par le retour élastique de l'élément de séparation (14) lors de la suppression au moins partielle de la force centrifuge.

15. Procédé selon la revendication 14, **caractérisé en ce que** le mélange (2) est exposé à la force centrifuge pendant une période d'au moins 10 min.
